# EUROPEAN PATENT APPLICATION

(11) **EP 4 563 697 A1**
(43) Date of publication of application: **04.06.2025**
(21) Application number: 23846472.1
(22) Date of filing: 24.07.2023
(51) Int. Cl.: C12N 5/075, C12N 5/071, C12N 5/10

(54) **IN VITRO CULTURING METHOD FOR INDUCING OVARIAN FOLLICLES FROM FETAL OVARIAN CELLS OF PRIMATE**

(30) Priority: 25.07.2022 JP 2022117665
(71) Applicant: Kyoto University, Kyoto-shi, Kyoto 606-8501 (JP)
(72) Inventor: SAITOU, Mitinori, Kyoto-shi, Kyoto 606-8501 (JP); OHTA, Hiroshi, Kyoto-shi, Kyoto 606-8501 (JP); MIZUTA, Ken, Kyoto-shi, Kyoto 606-8501 (JP)
(74) Representative: Hoffmann Eitle
(86) International application number: PCT/JP2023/027054
(87) International publication number: WO 2024/024742

(57) **Abstract**

The present invention provides a method for producing oocytes or follicles, or a reconstituted ovary containing oocytes or follicles, the method comprising a step for culturing a reconstituted ovary of a primate under a floating culture condition.

## Description

### TECHNICAL FIELD

The present invention relates to a method for inducing ovarian follicles or the like *in vitro.* In more detail, the present invention relates to a method for producing oocytes or ovarian follicles, or a reconstituted ovary containing oocytes or ovarian follicles, the method comprising a step of culturing the reconstituted ovary in a floating culture.

### BACKGROUND ART

Germ cells differentiate into either ova or sperm, which combine to form a zygote with full developmental capacity, thereby ensuring the persistence and diversity of genetic information across generations. Disruption of germ cell development can have serious consequences, including infertility and genetic or epigenetic disorders in offspring. Understanding mechanisms of germ cell development is therefore fundamental topic in both biology and medicine.

In mammals, germ cells arise early during embryonic development as primordial germ cells (PGCs). PGCs initiate epigenetic reprogramming, undergo migration, and colonize in the embryonic gonad. In the embryonic gonads, PGCs, called oogonia in females and gonocytes in males, exhibit similar genetic and epigenetic properties until they initiate overt sexual differentiation. In females, in response to cues from fetal ovaries, in particular, the pre-granulosa cells, oogonia differentiate into oocytes with rapid entry in the early meiotic prophase, oocytes that have completed the first meiotic prophase are surrounded by a single layer of granulosa cells to form primordial follicles that function as the source of oogenesis. In contrast, in males, in response to cues from the fetal testis, in particular, Sertoli cells, gonocytes differentiate into pro-spermatogonia and then into spermatogonia/spermatogonial stem cells (SSCs) that function as the source of spermatogenesis. The mechanisms of mammalian germ cell development have been intensively studied using mice as a model organism, and with the findings gained from such studies and advances in pluripotent stem cells (PSCs) and reproductive technologies, the entire process of both female and male germ cell development in mice has been reconstituted *in vitro* using mouse PSCs (mPSCs) as a starting substance.

For example, it has been reported that ovarian follicles can be induced by aggregating primordial germ cell-like cells (PGCLCs), which are produced through inducing differentiation from mouse pluripotent stem cells, with fetal ovarian somatic cells to produce a reconstituted ovary, and culturing the reconstituted ovary using the air-liquid interface culture method (Non Patent Literature 1). Similarly, it has been reported that ovarian follicles can also be induced by culturing fetal ovary tissues using the air-liquid interface culture method (Non Patent Literature 2).

The techniques for producing reconstituted ovaries in mice can also be applied to human PGCLCs. The inventors of the present invention previously constructed cross-species reconstituted ovaries by aggregating human PGCLCs and mouse fetal ovarian somatic cells, and successfully differentiated the reconstituted ovaries into early oocyte-like cells at the initiation of the first meiotic division by culturing the reconstituted ovaries for about three months using the air-liquid interface culture method (Non Patent Literature 3). However, there is no known method for long-term culture of allogeneic reconstituted ovaries of primates, including humans, and there is no *in vitro* culture method for inducing cells derived from fetal ovaries of primates to ovarian follicles (primordial follicles/primary ovarian follicles) having more mature structures.

### CITATION LIST

### NON PATENT LITERATURE

NON PATENT LITERATURE 1: Hayashi K, et al., Nat Protoc. 12(9): 1733-1744 (2017)
NON PATENT LITERATURE 2: Morohaku K, et al., Nat Protoc. 12(9): 1817-1829 (2017)
NON PATENT LITERATURE 3: Yamashiro C, et al., Science. 362(6412): 356-360 (2018)

### SUMMARY OF INVENTION

### TECHNICAL PROBLEM

Therefore, it is an object of the present invention to provide a method for maintaining reconstituted ovaries of primates, including humans, in an *in vitro* culture environment for a long period of time (for example, 7 weeks or more, 9 weeks or more, preferably 12 weeks or more). Also, it is another object of the present invention to provide a method for reproducing progression of meiosis and formation of ovarian follicles within the reconstituted ovaries by culturing the reconstituted ovaries for a long period of time.

### SOLUTION TO PROBLEM

The inventors made attempts to identify conditions under which reconstituted ovaries of primates, including humans, can be cultured *in vitro* for long periods of time. First, cynomolgus monkeys were used as a primate model to collect fetal ovaries from the cynomolgus monkeys, the cells were subjected to tissue dissociation treatment using enzymes or the like and cryopreserved (long-term), the cells were thawed, and then reconstituted ovaries (cell aggregates) were produced. The inventors cultured the produced cynomolgus monkey reconstituted ovaries (cy reconstituted ovaries) using a conventional air-liquid interface culture method, and found that the reconstituted ovaries gradually collapsed as the culture progressed and it was difficult to maintain their morphology. In other words, it became clear that the conventional air-liquid interface culture method is not suitable for long-term culture of cy reconstituted ovaries *in vitro.* It is conceivable that the difference between mice and cynomolgus monkeys reflects differences in biophysical properties of mouse and primate ovarian cells.

In view of this, the inventors examined conditions suitable for long-term culture of reconstituted ovaries of primates. As a result of intensive research conducted, the inventors concluded that a main advantage of the air-liquid interface culture method is improvement of aeration and oxygen supply to the cultured tissues, but that aeration and oxygen supply may not be considered important in the culture of mammalian ovaries. In view of this, the inventors decided to adopt a completely different culture method instead of improving the air-liquid interface culture method. Specifically, the inventors focused on a floating culture method, and revealed that by actually culturing cynomolgus monkey reconstituted ovaries using this method, the reconstituted ovaries can be maintained for a long period of time of 12 weeks or more, and further revealed that, through the culture, the mitotic oogonia in the reconstituted ovaries differentiate into oocytes, which completes the meiotic prophase I, and the reconstituted ovaries contain primordial follicle-like structures. Analysis of cell morphology and the expression/intracellular localization of key markers in these cells, using multiple analytical techniques such as transcriptome analysis, confirmed that the primordial follicle-like structures obtained through the above culture have properties extremely similar to those of *in vivo* primordial follicles. Furthermore, the inventors found that this *in vitro* culture method is similarly applicable to human fetal ovarian cells. The inventors conducted further studies based on these findings and have achieved the present invention.

That is to say, the present invention is as follows.
[1] A method for producing oocytes or ovarian follicles, or a reconstituted ovary containing oocytes or ovarian follicles, comprising a step of culturing a reconstituted ovary of a primate under a floating culture condition.
[2] The method according to [1], wherein the reconstituted ovary is constructed by aggregating primate germ cells and primate fetal ovarian somatic cells.
[3] The method according to [2], wherein the germ cells and the fetal ovarian somatic cells are prepared by dissociation from a fetal ovary of the primate through enzymatic treatment.
[4] The method according to [2] or [3], wherein the germ cells are at least one type selected from the group consisting of oogonia, primordial germ cells, and primordial germ cell-like cells.
[5-1] The method according to any one of [2] to [4], wherein at least either one of the germ cells and the ovarian somatic cells are derived from pluripotent stem cells.
[5-2] The method according to [5-1], wherein the pluripotent stem cells are induced pluripotent stem cells.
[6-1] The method according to any one of [1] to [5-2], wherein the culture is carried out under the floating culture condition for 9 weeks or more.
[6-2] The method according to any one of [1] to [5-2], wherein the culture is carried out under the floating culture condition for 12 weeks or more.
[7-1] The method according to any one of [1] to [6-2], wherein the culture under the floating culture condition is a culture in a medium containing L-glutamine, ascorbic acid, and 2-mercaptoethanol.
[7-2] The method according to [7-1], wherein the concentration of L-glutamine in the medium is 10 µM to 1 M.
[7-3] The method according to [7-1] or [7-2], wherein the concentration of ascorbic acid in the medium is 1 µM to 1 M.
[7-4] The method according to any one of [7-1] to [7-3], wherein the concentration of 2-mercaptoethanol in the medium is 0.1 µM to 1 M.
[8] The method according to any one of [7-1] to [7-4], wherein the medium further contains retinoic acid.
[9-1] The method according to any one of [7-1] to [8], wherein the medium further contains one or more selected from the group consisting of progesterone, cortisol, dehydroepiandrosterone, and dehydroepiandrosterone sulfate.
[9-2] The method according to [9-1], wherein the concentration of retinoic acid in the medium is 0.1 nM to 5 µM.
[9-3] The method according to [9-1] or [9-2], wherein the concentration of progesterone in the medium is 0.01 µg/mL to 10 µg/mL.
[9-4] The method according to any one of [9-1] to [9-3], wherein the concentration of cortisol in the medium is 0.01 µg/mL to 10 µg/mL.
[9-5] The method according to any one of [9-1] to [9-4], wherein the concentration of dehydroepiandrosterone in the medium is 0.1 nM to 5 µM.
[9-6] The method according to any one of [9-1] to [9-5], wherein the concentration of dehydroepiandrosterone sulfate in the medium is 0.1 µM to 5 mM.
[9-7] The method according to any one of [7-1] to [9-6], wherein the medium contains serum.
[9-8] The method according to [9-7], wherein the concentration (v/v) of serum in the medium is 0.01% to 50%.
[10] The method according to any one of [1] to [9-8], wherein the primate is a human.
[11] Oocytes, ovarian follicles, or a reconstituted ovary produced by the method according to any one of [1] to [10].
[12] A method for producing an ovum, comprising a step of maturing the oocytes according to [11].
[13-1] A kit for producing oocytes or ovarian follicles, or a reconstituted ovary containing oocytes or ovarian follicles, comprising a culture vessel for floating culture, L-glutamine, ascorbic acid, and 2-mercaptoethanol.
[13-2] The kit according to [13-1], further comprising one or more molecules selected from the group consisting of retinoic acid, progesterone, cortisol, dehydroepiandrosterone, and dehydroepiandrosterone sulfate.
[13-3] The kit according to [13-1] or [13-2], further comprising serum.
[14] The kit according to any one of [13-1] to [13-3], further comprising a basal medium.
[15-1] The kit according to any one of [13-1] to [14], further comprising retinoic acid.
[15-2] The kit according to any one of [13-1] to [15-1], further comprising one or more selected from the group consisting of progesterone, cortisol, dehydroepiandrosterone, and dehydroepiandrosterone sulfate.

### ADVANTAGEOUS EFFECTS OF INVENTION

With the present invention, it is possible to maintain reconstituted ovaries of general primates, including humans, *in vitro* for a long period of time (for example, 7 weeks or more, 9 weeks or more, preferably 12 weeks or more), and to mature germ cells such as primordial germ cell-like cells, primordial germ cells, and oogonia into meiotic cells, thus inducing primordial follicle-like structures. By using such a method and the primordial follicle-like structures induced using the method, it is possible to observe the differentiation process of primate female germ cells *in vitro,* which is useful for understanding the mechanism of germ cell development. Thus, the present invention provides an important foundation for both biology and medicine.

### BRIEF DESCRIPTION OF DRAWINGS

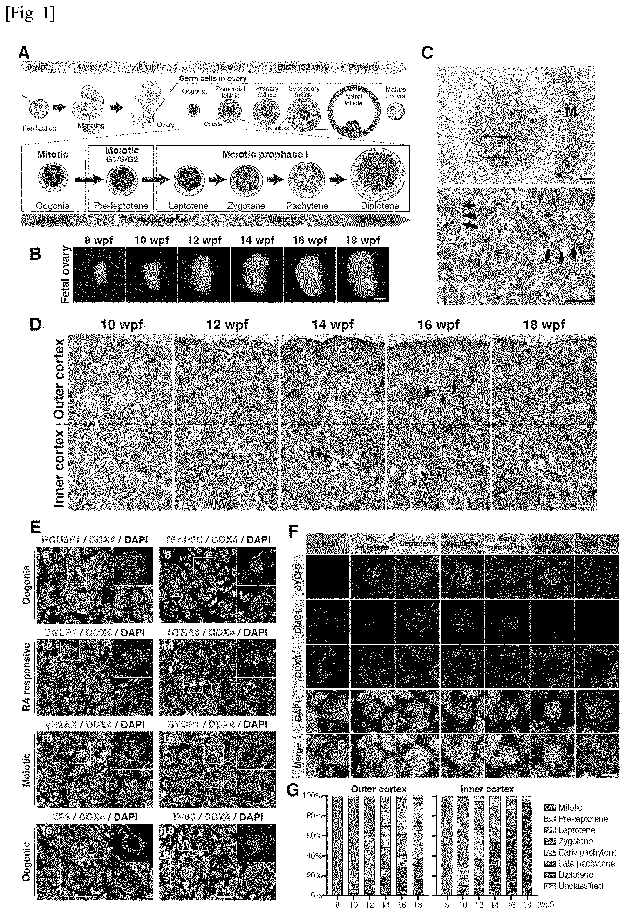

[Fig. 1] Fetal ovary development in cynomolgus monkeys. (A) Scheme of female germ cell development in cynomolgus monkeys (cy). Wpf, weeks post-fertilization; PGCs, primordial germ cells. (B) Gross appearances of developing cy ovaries at 8-18 wpf. Scale bar = 1 mm. (C) Hematoxylin and eosin (H&E) staining of a cy fetal ovary at 8 wpf. Arrows indicate oogonia. M, mesonephros. Scale bars = 100 µm (top), 40 µm (bottom). (D) H&E staining of the ovarian cortex in cy fetuses at 10-18 wpf. The ovarian cortex was divided into the outer and inner cortex at the line bisecting the ovarian cortex. Black arrows, oocytes bearing a large nucleus with condensed, thread-like chromatin; white arrows, primordial follicles. Scale bar = 50 µm. (E) Immunofluorescence (IF) analysis for oogonia (POU5F1, TFAP2C), RA responsive (ZGLP1, STRA8), meiotic (γH2AX, SYCP1), and oogenic (ZP3, TP63) markers during cy fetal ovary development. Representative images for each marker are shown. Germ cells were marked with DDX4. Nuclear DAPI staining is shown in white. The upper-right magnified images of each panel show the expression of key markers co-stained with DDX4. The lower-right magnified images of each panel show DDX4 expression co-stained with DAPI (white). The numbers written in the upper-left corner indicate the stage (wpf) of the fetus. Scale bar = 20 µm. (F) Representative images of germ cells immunostained for SYCP3, DMC1, and DDX4 at each meiotic sub-stage. Nuclear DAPI staining is shown in white. The sub-stages of meiosis were defined based on the staining patterns of SYCP3, DMC1, and DAPI as described in <Materials and Methods>. Scale bar = 10 µm. (G) Percentages of germ cells at each meiotic sub-stage as defined in Fig. 1F. The average percentages of outer or inner ovarian cortex on each meiotic sub-stage were calculated from at least three fetal ovaries. The color-coding is as indicated.

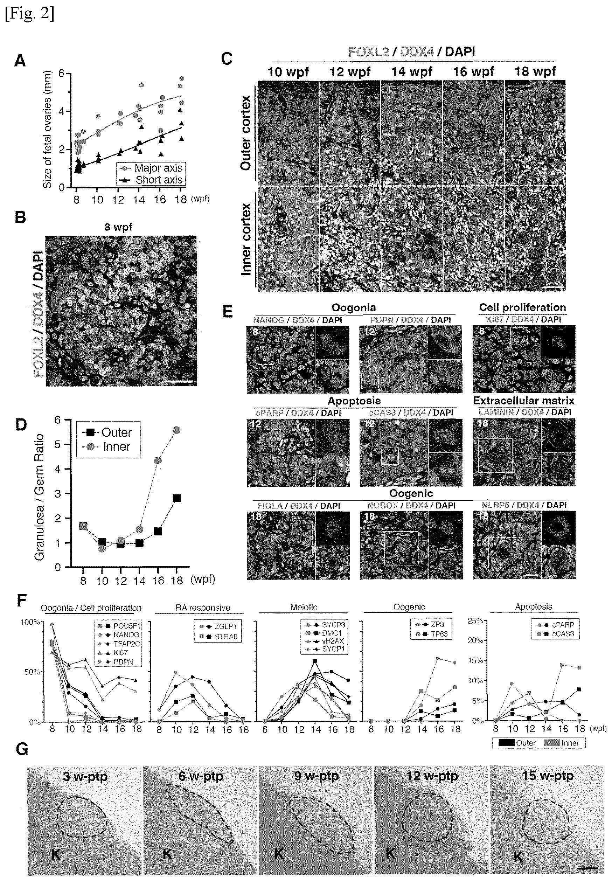

[Fig. 2] Histological analyses of cy fetal ovaries *in vivo* and in xenotransplanted reconstituted ovaries. (A) Size of developing cy fetuses and ovaries at 8-18 wpf. Lines indicate LOESS curves fitted to the sample populations. (B and C) IF of a cy fetal gonad at 8 wpf (B) and ovarian cortexes in cy fetuses at 10-18 wpf (C). The results of staining with FOXL2 (granulosa cell marker), DDX4 (germ cell marker), and DAPI (nucleus, white) are shown. The ovarian cortexes at 10-18 wpf were divided into an outer and inner cortex at the line bisecting the ovarian cortex. Scale bars = 40 µm (B) and 50 µm (C). (D) The granulosa/germ cell ratios at each developmental stage assessed from the IF data are shown. (E) IF for cell proliferation (Ki67), apoptosis (cleaved PARP, cleaved CASPASE 3), extracellular matrix (LAMININ), and oogenic (FIGLA, NOBOX, NLRP5) markers during cy fetal ovary development. Representative images for each marker are shown. Germ cells were marked with DDX4. Nuclear DAPI staining is shown in white. The upper-right magnified images of each panel show the expression of key markers co-stained with DDX4 except for NLRP5. The lower-right magnified images of each panel show DDX4 expression co-stained with DAPI (white). The numbers written in the upper-left corner indicate the stage (wpf) of the fetus. Scale bar = 20 µm. (F) Percentages of cells positive for individual markers among DDX4⁺ germ cells from the IF of the cy fetal ovarian outer/inner cortex at 8-18 wpf. The mean values from more than three biological replicates are shown. (G) H&E staining of transplanted cy reconstituted ovaries at 3/6/9/12/15 weeks post-transplantation (w-ptp). Dashed lines indicate the transplanted cy reconstituted ovaries beneath the kidney capsule of KSN/Slc mice. K, mouse kidney. Scale bar = 200 µm.

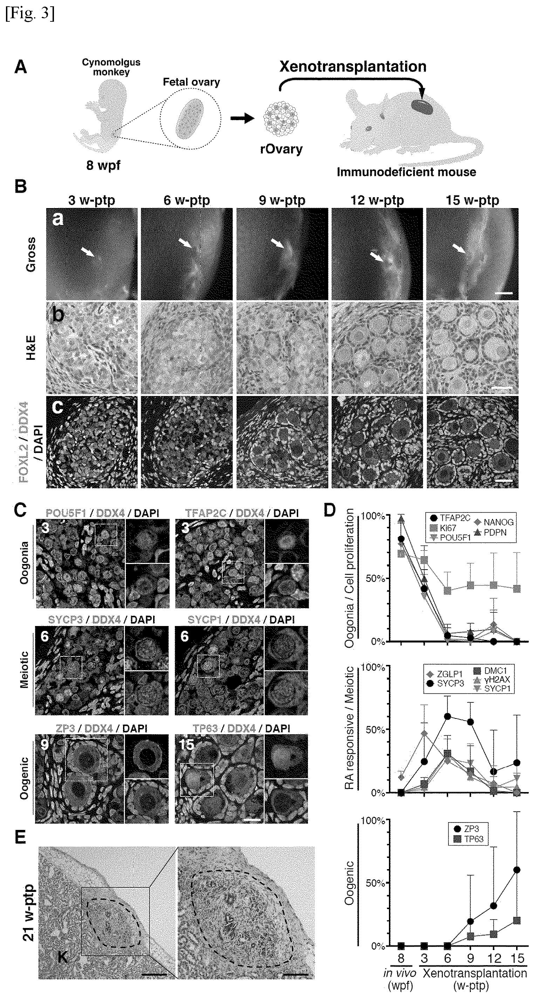

[Fig. 3] Derivation of cy ovarian follicles from fetal gonadal cells by xenotransplantation. (A) Scheme of the xenotransplantation experiment. The cy reconstituted ovaries generated from *in vivo* ovarian cells at 8 wpf were transplanted beneath the kidney capsule of immunodeficient KSN/Slc mice (see <Materials and Methods>). (B) Gross appearances (a), H&E staining (b), and IF for FOXL2/DDX4/DAPI (c) of transplanted cy reconstituted ovaries at 3/6/9/12/15 weeks post-transplantation (w-ptp). The primordial follicle-like complexes surrounded with FOXL2⁺ granulosa cells were observed inside the reconstituted ovaries at 12 and 15 w-ptp. Arrows indicate the reconstituted ovaries. FOXL2, granulosa cell marker; DDX4, germ cell marker. Scale bars = 1 mm (a), 40 µm (b and c). (C) IF for oogonia (POU5F1, TFAP2C), meiotic (SYCP3, SYCP1), and oogenic (ZP3, TP63) markers in the transplanted cy reconstituted ovaries. Germ cells and nuclei were marked with DDX4 and DAPI (white), respectively. The upper-right magnified images of each panel show the expression of each marker co-stained with DDX4. The lower-right magnified images of each panel show DDX4 expression co-stained with DAPI (white). The numbers written in the upper-left corner indicate weeks after transplantation (w-ptp). Scale bar = 20 µm. (D) Percentages of cells positive for individual markers among DDX4⁺ germ cells from the IF of cy reconstituted ovaries at 3-15 w-ptp. The average values with SDs are shown. (E) H&E staining of transplanted cy reconstituted ovaries at 21 w-ptp. Dashed lines indicate the transplanted cy reconstituted ovaries beneath the kidney capsule of KSN/Slc mice. K, mouse kidney. Scale bars = 200 µm (left) and 100 µm (right).

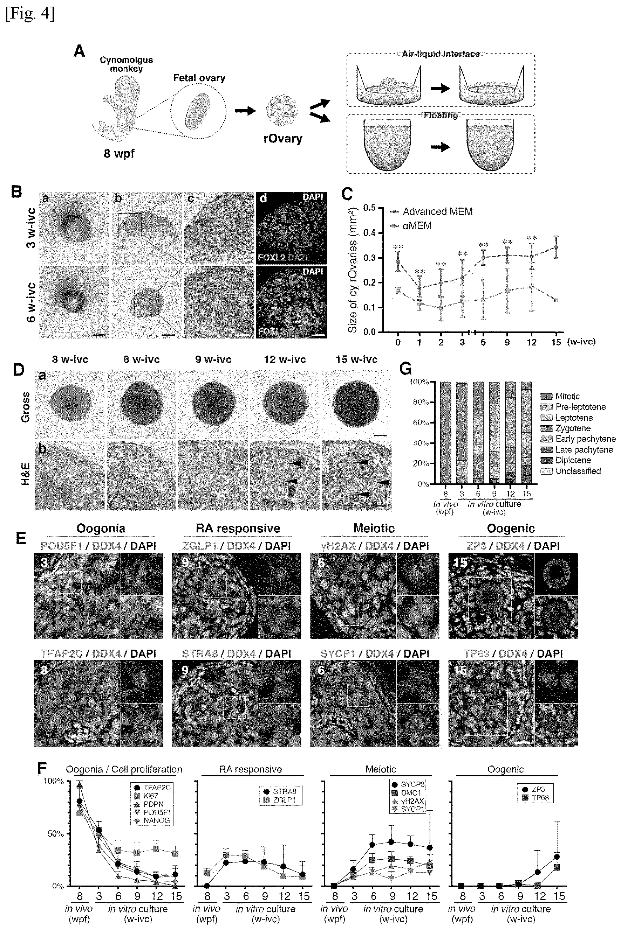

[Fig. 4] *In vitro* culture system for inducing ovarian follicles from fetal gonadal cells. (A) Scheme of the *in vitro* culture system for inducing cy ovarian follicles. The cy reconstituted ovaries generated from fetal gonadal cells at 8 wpf were cultured in either an air-liquid interface or a floating condition (see <Materials and Methods>). (B) Gross appearance (a), H&E staining (b, c), and IF for FOXL2/DAZL/DAPI (d) of cy reconstituted ovaries cultured in the air-liquid interface condition at 3 and 6 weeks of *in vitro* culture (w-ivc). FOXL2, granulosa cell marker; DAZL, germ cell marker; DAPI, nucleus. Scale bars = 200 µm (a), 100 µm (b), and 40 µm (c and d). (C) Quantitative analysis for the size of cy reconstituted ovaries cultured in Advanced MEM or αMEM as the basal media of P-IVD medium. The mean values from more than three biological replicates are shown with SDs except for the reconstituted ovaries in αMEM at 15 w-ive (n=1). **P < 0.01, Welch's T-test. (D) Gross appearance (a) and H&E staining (b) images of cy reconstituted ovaries cultured with the P-IVD medium at 3/6/9/12/15 w-ivc. Arrowheads indicate primordial follicle-like structures. Scale bars = 200 µm (a) and 40 µm (b). (E) IF analyses of key markers as described in Fig. 1E in the cultured cy reconstituted ovaries. Germ cells and nuclei were marked with DDX4 and DAPI (white), respectively. The upper-right magnified images of each panel show the expression of key markers co-stained with DDX4. The lower-right magnified images of each panel show DDX4 expression co-stained with DAPI (white). The numbers written in the upper-left corner indicate the period of IVC (w-ivc). Scale bar = 20 µm. (F) Percentages of cells positive for individual markers among DDX4⁺ germ cells from the IF of cy reconstituted ovaries at 3-15 w-ivc. The average values with SDs are shown. (G) Percentages of germ cells at each meiotic sub-stage as defined in Fig. 1F (see also <Materials and Methods>). The average percentages of each meiotic sub-stage were determined from at least three cy reconstituted ovaries.

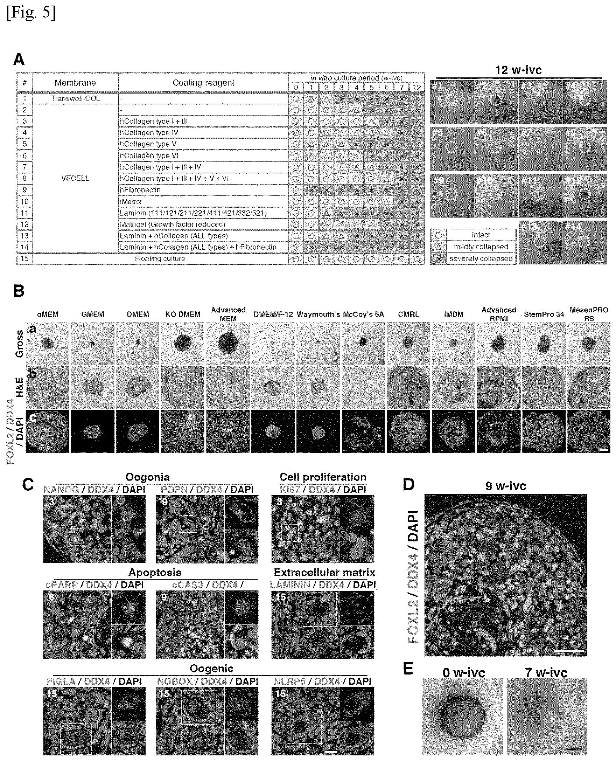

[Fig. 5] Establishment of a novel *in vitro* culture system for primate oocyte differentiation. (A) (Left) Summary table of coating conditions tested for cy reconstituted ovaries. (Right) Representative images of cy reconstituted ovaries cultured on membranes at 12 w-ivc. Dotted circles show the edge of cy reconstituted ovaries at the starting point of air-liquid interface culture. Scale bar = 500 µm. (B) Gross appearances (a), H&E staining (b), and IF for FOXL2/DDX4/DAPI (c) of cy reconstituted ovaries cultured in 13 different basal media at 6 w-ivc. The reconstituted ovary cultured in Advanced MEM maintained its size, and DDX4⁺ germ and FOXL2⁺ granulosa cells were found within the reconstituted ovaries. Scale bars = 500 µm (a) and 40 µm (b and c). (C) IF analyses of key markers as described in Fig. 2E in the cultured cy reconstituted ovaries. Germ cells and nuclei were marked with DDX4 and DAPI (white), respectively. The upper-right magnified images of each panel show the expression of key markers co-stained with DDX4 except for NLRP5. The lower-right magnified images of each panel show DDX4 expression co-stained with DAPI (white). The numbers written in the upper-left corner indicate the period of IVC (w-ivc). Scale bar = 20 µm. (D) IF analysis for FOXL2 (granulosa cells)/DDX4 (germ cells) stained with DAPI (nucleus) in the cultured cy reconstituted ovary at 9 w-ivc. Scale bar = 40 µm. (E) Representative images of human reconstituted ovaries cultured on Transwell-COL membranes at 0 and 7 w-ivc. Scale bar = 200 µm.

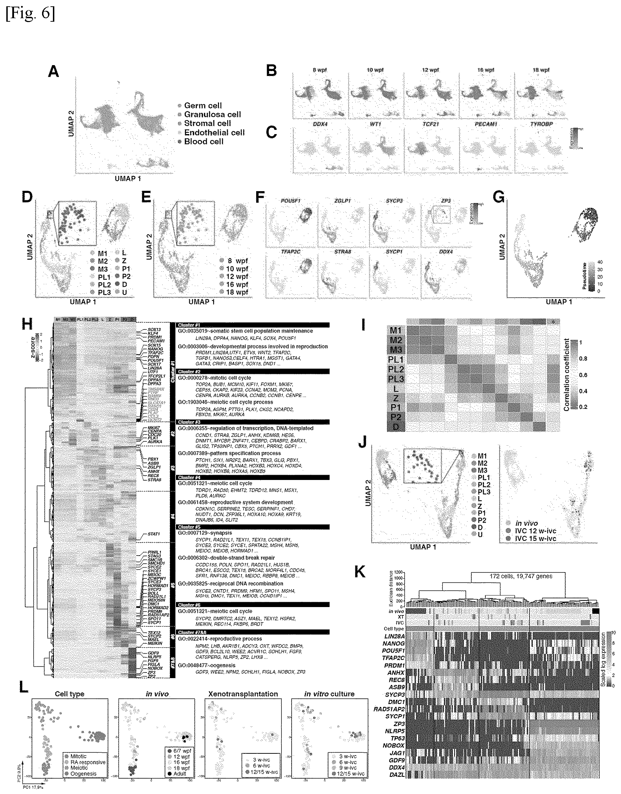

[Fig. 6] Single-cell RNA-seq analyses of cy ovarian cells *in vivo* and in reconstituted ovaries. (A-J) Analyses for cy germ cells by 10X scRNA-seq. (A) Uniform manifold approximation and projection (UMAP) plot of cy fetal ovarian cells *in vivo,* colored by five computationally assigned major clusters based on the expression of cell-type-specific markers. The color-coding is as indicated. (B) UMAP plot shown in Fig. 6A, the cells for each fetal stage are shown in black. (C) Feature plots for the expression of key marker genes for cy *in vivo* germ cells (DDX4), granulosa cells (WT1), stromal cells (TCF21), endothelial cells (PECAM1), and blood cells (TYROBP) overlaid on the UMAP plot in Fig. 6A. (D) UMAP plot of cy *in vivo* germ cells shown with the clustering of meiotic sub-stages. The color-coding is as indicated. M, mitotic; PL, pre-leptotene; L, leptotene; Z, zygotene; P, pachytene; D, diplotene; U, unclassified. (E) UMAP plot of cy *in vivo* germ cells colored by the fetal stage. The color-coding is as indicated. The color-coding is as indicated. (F) Gene expression plots for oogonia (POU5F1, TFAP2C), RA-responsive (ZGLP1, STRA8), meiotic (SYCP3, SYCP1), oogenic (ZP3), and germ-cell (DDX4) markers during cy *in vivo* germ-cell development. (G) UMAP plot of cy *in vivo* germ cells colored by pseudotime. The color-coding for pseudotime is as indicated. (H) (Left) Heatmap of the standardized expression of highly variable genes (HVGs, 1,481 genes) among *in vivo* cy germ cell sub-stages ordered by unsupervised hierarchical clustering (UHC); eight gene clusters were defined according to the UHC dendrogram. Key genes for female germ cell development are shown in black, and 10 genes (TMSB4X, PTRF, BAMBI, RND3, SLC47A1, RASD1, CD24, PLAT, CCL3 and GCNT2) transiently upregulated in the PL cluster are shown in gray. (Right) Representative genes and key gene ontology (GO) enrichments are shown. (I) Heatmap of the Pearson correlation coefficients of the average expression levels of the 1,481 HVGs among the meiotic sub-stages in cy *in vivo* germ cells. The asterisk indicates an unclassified cluster. The color-coding is as indicated. (J) (Left) UMAP plot for germ cells from cy *in vivo* fetal ovaries and *in vitro* cultured cy reconstituted ovaries, shown with the meiotic sub-stages. The color-coding is as indicated. (Right) UMAP plot highlighting cells from *in vitro* cultured cy reconstituted ovaries at 12 and 15 w-ivc. (K and L) Analyses for cy germ cells by SC3-seq. (K) UHC for cy germ cells *in vivo* and in reconstituted ovaries with all expressed genes (19,747 genes) and a heatmap of the expression levels of selected marker genes. Color bars under the dendrogram show the *in vivo* stage (1^{st} bar), the period of xenotransplantation (2^{nd} bar), the period of IVC (3^{rd} bar), and the cell type (4^{th} bar). The heatmap color-coding is as indicated. The color-coding of bars is as indicated in Fig. 6L. (L) Principal component analysis (PCA) of cy germ cells *in vivo* and in reconstituted ovaries performed by SC3-seq. The cells were plotted in the two-dimensional plane defined by PC1 and PC2 values. The color-coding is as indicated.

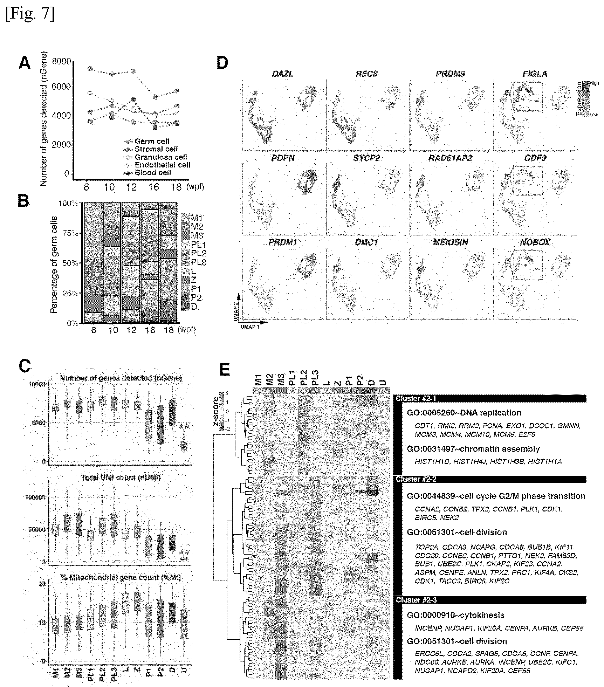

[Fig. 7] 10X scRNA-seq analysis of cy female oocyte development *in vivo.* (A) The average number of genes detected in five major cell types in cy *in vivo* fetal ovaries at 8-18 wpf. (B) Percentages of germ cells at the indicated meiotic prophase sub-stages defined in Fig. 6D. The color-coding is as indicated. M, mitotic; PL, pre-leptotene; L, leptotene; Z, zygotene; P, pachytene; D, diplotene. (C) The detected gene number, UMI count, and percentage of mitochondrial genes in each germ cell sub-stage. The number of genes and UMI counts were significantly low in the "unclassified (U)" compared to any other germ cell cluster. **P < 0.01, Tukey-Kramer tests. (D) Feature plots of key marker genes for oocyte development on the UMAP plot shown in Fig. 6D. (E) (Left) Heatmap of the standardized expression of HVGs listed in the gene cluster #2 in Fig. 6H. Three gene clusters (Cluster #2-1/-2/-3) were defined according to the UHC dendrogram. (Right) Representative genes and key GO enrichments are shown.

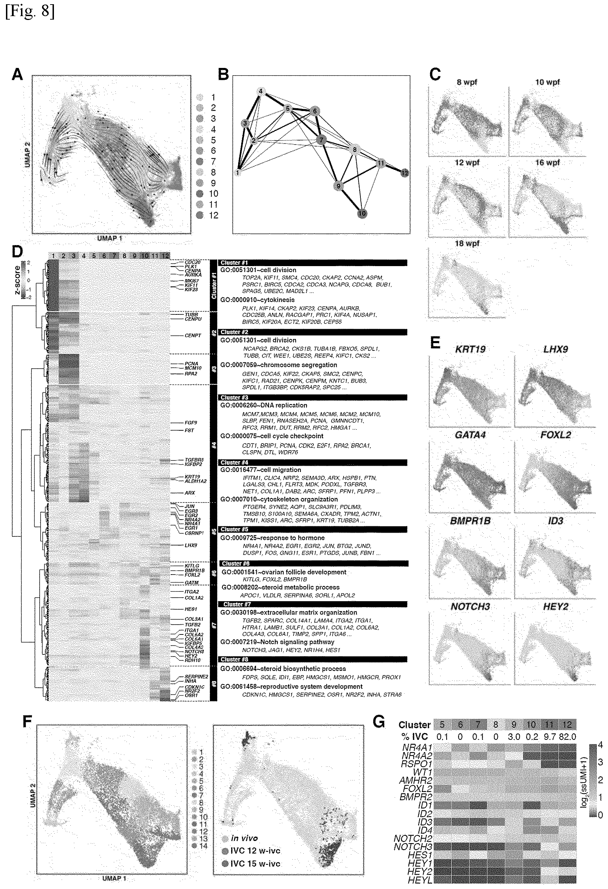

[Fig. 8] 10X scRNA-seq analysis for cy granulosa cells *in vivo* and in reconstituted ovaries. (A and B) UMAP with RNA velocity (A) and PAGA graph (B) of cy *in vivo* granulosa cells as defined in Fig. 6A. Granulosa cells were divided into 12 sub-clusters by Louvain clustering. The color-coding is as indicated. (C) In the UMAP plot shown in Fig. 8A, the cells for each fetal stage are shown in black. (D) (Left) Heatmap of the standardized expression of HVGs (712 genes) among *in vivo* cy granulosa cell sub-clusters ordered by UHC; eight gene clusters were defined according to the UHC dendrogram. (Right) Representative genes and key GO enrichments are shown. (E) Feature plots of key marker genes for cy granulosa cell development on the UMAP plot shown in Fig. 8A. (F) (Left) UMAP plot for granulosa cells from cy *in vivo* fetal ovaries and *in vitro* cultured cy reconstituted ovaries, shown with the granulosa sub-clusters. The color-coding is as indicated. (Right) The UMAP plot highlighting cells from *in vitro* cultured cy reconstituted ovaries at 12 and 15 w-ivc. (G) Heatmap of the average expression levels of granulosa cell marker, BMP signaling pathway (BMPR2 and ID1/2/3/4), and NOTCH signaling pathway (NOTCH2/3, HES1, and HEY1/2/L) genes in granulosa cell sub-clusters #5-12 defined in Fig. 8F. The percentages of cells derived from cultured reconstituted ovaries in each sub-cluster are shown (%IVC). The color-coding is as indicated.

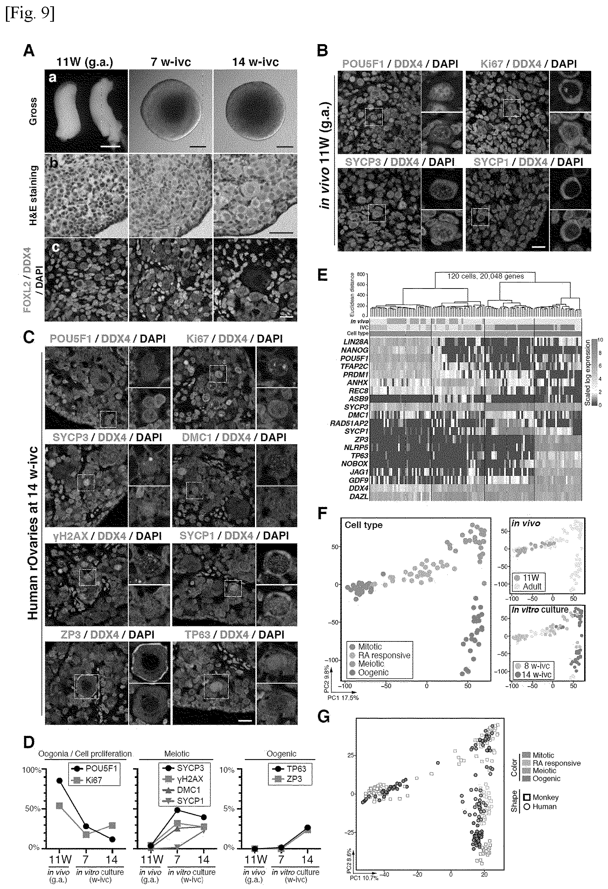

[Fig. 9] Derivation of human ovarian follicles from human oogonia *in vitro.* (A) Gross appearance (a), H&E staining (b), and IF (c) for FOXL2/DDX4/DAPI of the human *in vivo* female gonads (11W, left column) and the cultured reconstituted ovaries at 7 and 14 w-ivc. FOXL2, granulosa cell marker; DDX4, germ cell marker; DAPI, nucleus. Scale bars = 1 mm (a, left), 200 µm (a, middle/right), 50 µm (b), and 20 µm (c). g.a., gestational age. (B and C) IF for oogonia (POU5F1), cell proliferation (Ki67), meiotic (SYCP3, DMC1, γH2AX, SYCP1), and oogenic (ZP3, TP63) markers for human *in vivo* female gonad (11W, B) and human *in vitro* cultured reconstituted ovaries at 14 w-ivc (C). Germ cells and nuclei were marked with DDX4 and DAPI (white), respectively. The upper right magnified images of each panel show the expression of key markers co-stained with DDX4. The lower right magnified images of each panel show DDX4 expression co-stained with DAPI (white). Scale bar = 20 µm. (D) Percentages of cells positive for individual markers among DDX4⁺ germ cells from the IF of human *in vivo* female gonads (11W) and human cultured reconstituted ovaries at 7 and 14 w-ivc. The mean values from two biological replicates are shown. (E and F) Analyses for human germ cells by SC3-seq. (E) UHC for human germ cells *in vivo* and in reconstituted ovaries with all expressed genes (20,048 genes) and a heatmap of the expression levels of selected marker genes. Color bars under the dendrogram show the *in vivo* stage (1^{st} bar), the period of IVC (2^{nd} bar), and the cell type (3^{rd} bar). The heatmap color-coding is as indicated. The color coding in bars is as indicated in Fig. 9F. (F) PCA of human germ cells *in vivo* and in reconstituted ovaries performed by SC3-seq. The cells are plotted in the two-dimensional plane defined by PC1 and PC2 values. The color-coding is as indicated. (G) PCA plot of all cy and human SC3-seq data merged by canonical correlation analysis (CCA). Cell types were defined after CCA followed by Louvain clustering. The color and shape coding are as indicated.

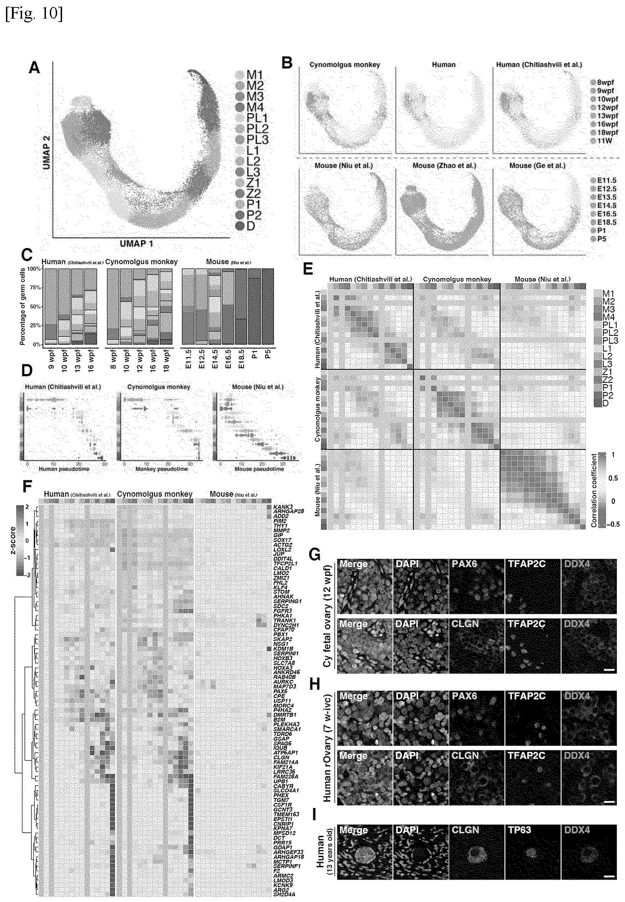

[Fig. 10] Cross-species comparison analysis of fetal female germ cell development *in vivo* in humans, monkeys, and mice. (A-F) Analyses performed by 10X scRNA-seq. (A) UMAP plot of germ cells from six 10X scRNA-seq datasets (two for humans [this Example and (Chitiashvili T, et al., Nat Cell Biol, 22: 1436-1446 (2020))], one for monkeys [this Example], and three for mice [(Ge W, et al., Cell Mol Life Sci, 78: 695-713 (2021), Niu W, and Spradling AC, Proc Natl Acad Sci USA, 117: 20015-20026 (2020), Zhao ZH, et al., FASEB J, 34: 12634-12645 (2020)]) integrated by CCA. Meiotic sub-stages were defined after CCA followed by Louvain clustering. Colors indicate the computationally assigned meiotic sub-stages defined by the expression levels of key meiotic markers conserved in all three species (see <Materials and Methods>). The color-coding is as indicated. M, mitotic; PL, pre-leptotene; L, leptotene; Z, zygotene; P, pachytene; D, diplotene. (B) The UMAP plot highlighting cells from each dataset. The color-coding for each developmental stage is as indicated. (C) Percentages of germ cells belonging to each meiotic sub-stage. The color-coding for the germ cell stage is as indicated in Fig. 10A. (D) The distribution of germ cells along the individually calculated species-specific pseudotime trajectories using Monocle. The color-coding for the germ cell stage is as indicated in Fig. 10A. (E) Heatmap of the Pearson correlation coefficients of the average expression levels of 237 HVGs (1-1-1 orthologues) among the meiotic sub-stages in humans, monkeys, and mice (see <Materials and Methods>). The color-coding for the germ cell stage is as indicated. (F) Heatmap of the standardized expression levels of selected genes using 10X scRNA-seq data in all three species. Genes showing specific expression changes in humans and monkeys, but not in mice, during female germ cell development were manually selected from the list of DEGs acquired from the SC3-seq analysis for cy/human *in vitro* cultured reconstituted ovaries. The heatmap color-coding is as indicated. The color-coding for the germ cell sub-stage is as indicated in Fig. 10E. (G and H) IF analyses of PAX6 and CLGN with TFAP2C (oogonia marker) and DDX4 (germ cell marker) in cy *in vivo* fetal ovaries at 12 wpf (G) and human cultured reconstituted ovaries at 7 w-ivc (H). Nuclear DAPI staining is shown in white. Scale bar = 20 µm. (I) IF analyses of CLGN with TP63 (oogenic marker) and DDX4 (germ cell marker) in a 13-year-old human oocyte of a primordial follicle. Nuclear DAPI staining is shown in white. Scale bar = 20 µm.

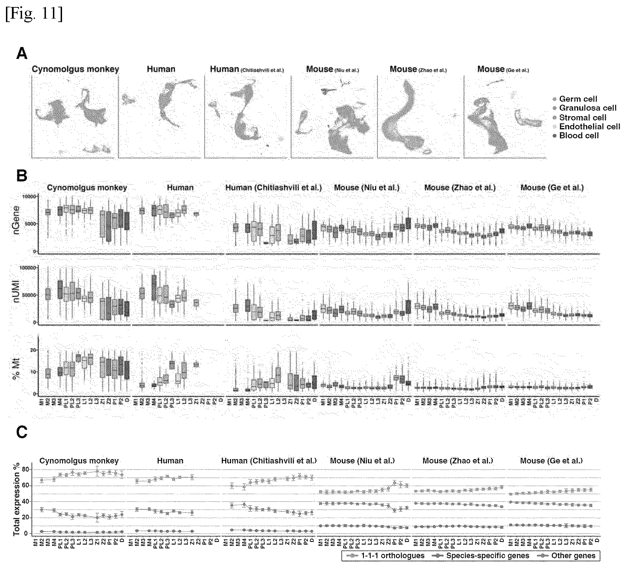

[Fig. 11] Cross-species analysis using 10X scRNA-seq datasets for *in vivo* fetal female germ cell development in humans, monkeys, and mice. (A) UMAP plots of fetal ovarian cells in each 10X scRNA-seq dataset, colored by five computationally assigned major clusters based on the expression of cell-type-specific markers. (B) The detected gene number (nGene), UMI count (nUMI), and percentage of mitochondrial genes (%Mt) in each germ cell meiotic sub-stage of six 10X scRNA-seq datasets. M, mitotic; PL, pre-leptotene; L, leptotene; Z, zygotene; P, pachytene; D, diplotene. (C) The percentages of average expression of gene classes (1-1-1 orthologues, species-specific, and other genes) during fetal oocyte development in three animal species are shown with SDs. The color-coding is as indicated.

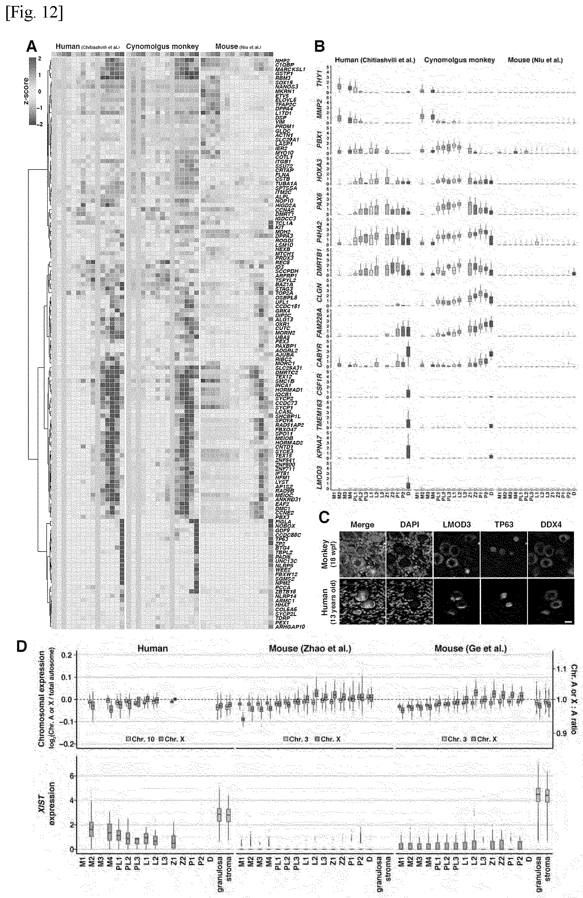

[Fig. 12] Cross-species comparison analysis of oocyte development in humans, monkeys, and mice. (A) Heatmap of the standardized expression levels of selected genes in all three species analyzed by 10X scRNA-seq. Genes showing a conserved expression pattern in all three animal species during female germ cell development were manually selected from the list of DEGs acquired from the SC3-seq analysis for cy/human *in vitro* cultured reconstituted ovaries. The color-coding is as indicated. (B) Box plots for the gene expression levels of selected markers showing primate-specific expression dynamics during fetal germ cell development (see also Fig. 10F). (C) IF analyses of LEIOMODIN-3 (LMOD3) with TP63 (oogenic marker) and DDX4 (germ cell marker) in cy *in vivo* fetal oocytes at 18 wpf (top) and 13-year-old human oocytes in primordial follicles (bottom). Nuclear DAPI staining is shown in white. Scale bar = 20 µm. (D) The autosome: A and X:A ratios (top) and XIST/Xist expression transitions (bottom) during female germ cell development *in vivo* in humans (this Example) and mice (Ge W, et al., Cell Mol Life Sci, 78: 695-713 (2021), Zhao ZH, et al., FASEB J, 34: 12634-12645 (2020)) analyzed by 10X scRNA-seq.

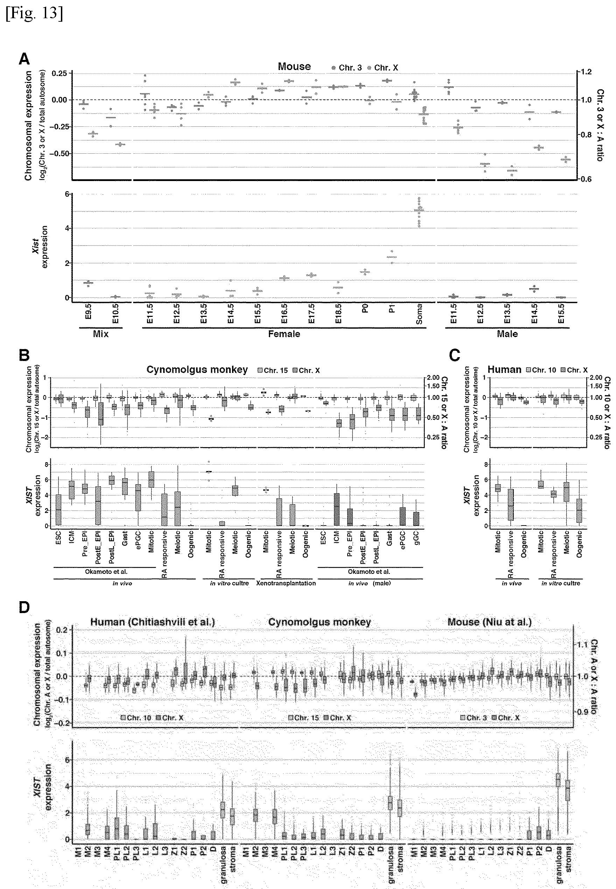

[Fig. 13] X:A ratios and XIST expression during germ-cell development in humans, monkeys, and mice. (A) The Chr.3:A and X:A ratios (top), and Xist expression (bottom) in mouse fetal/neonatal gonadal cells analyzed by bulk RNA-seq. Dot, each datum; bar, mean. Samples at E9.5/10.5 include germ cells from male and female embryos (mix). E, embryonic day; P, postnatal day; Soma, gonadal somatic cells. (B) The Chr.15: A and X:A ratios (top), and XIST expression (bottom) in cy female germ cells *in vivo* and in cultured/transplanted reconstituted ovaries analyzed by SC3-seq. The Chr.15:A/X:A ratio transitions in the embryonic and hypoblast lineages reported in (Okamoto I, et al., Science, 374: eabd8887 (2021)) are also shown. ESC, embryonic stem cell; ICM, inner cell mass; Pre_EPI, pre-implantation epiblast; PostE_EPI, post-implantation early epiblast; PostL_EPI, post-implantation late epiblast; Gast, gastrulating cells; ePGC, early primordial germ cells. Data with small sample size (n≤3) are indicated with dots (each datum) and bars (mean). (C) The Chr.10:A and X:A ratios (top), and XIST expression (bottom) in human female germ cells *in vivo* and in cultured reconstituted ovaries analyzed by SC3-seq. (D) The autosome:A and X:A ratios (top), and XIST/Xist expression transitions (bottom) during female germ cell development *in vivo* in all three species analyzed by 10X scRNA- seq.

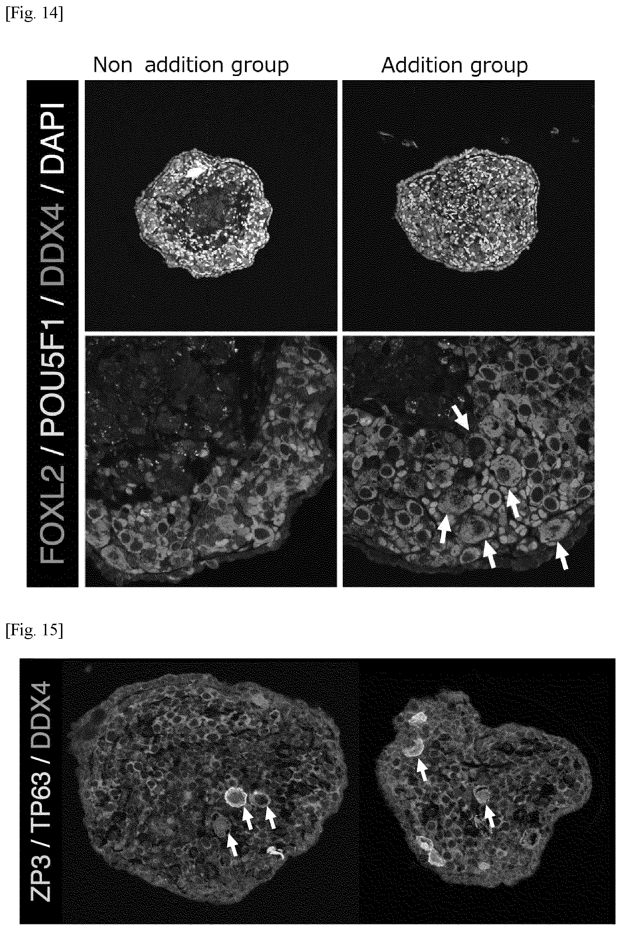

[Fig. 14] Histology of a reconstituted ovary at 9 weeks of *in vitro* culture. The formation of primordial follicles (arrows) was observed only in the steroid hormone addition group. FOXL2, granulosa cell marker; POU5F1, oogonia marker; DDX4, germ cell marker; DAPI, nucleus.
[Fig. 15] Histology of a reconstituted ovary at 9 weeks of *in vitro* culture. The formation of primordial follicles (arrows) was observed in the steroid hormone addition group. ZP3 and TP63, oocyte markers at ovarian follicle formation stage; DDX4, germ cell marker.

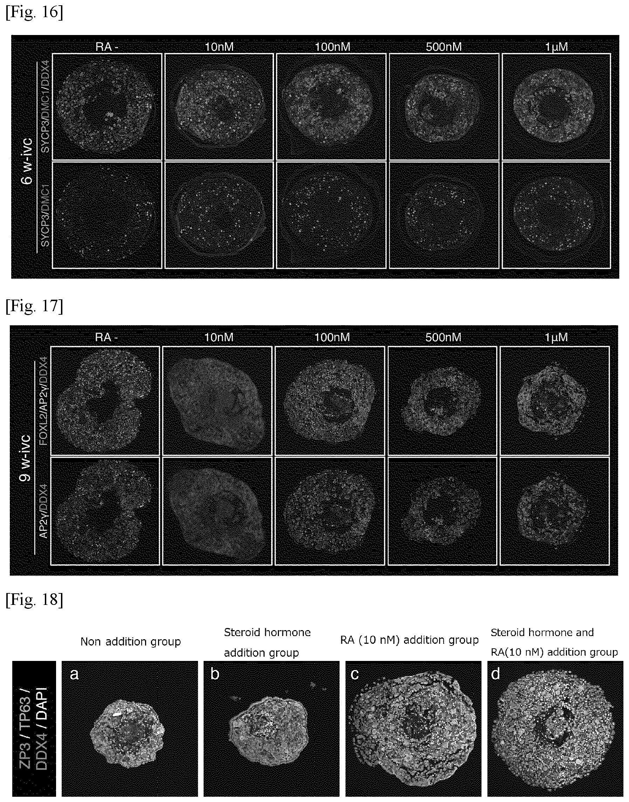

[Fig. 16] Histology of a reconstituted ovary at 6 weeks of *in vitro* culture (6 w-ivc). In the RA addition group, the SYCP3/DMC1 positive rate is high at all concentrations, and meiotic entry was facilitated. SYCP3 and DMC1, meiotic markers; DDX4, germ cell marker.
[Fig. 17] Histology of a reconstituted ovary at 9 weeks of *in vitro* culture (9 w-ivc). In the RA addition group, the AP2γ positive rate is low at all concentrations, and meiotic entry was facilitated. FOXL2, granulosa cell marker; AP2γ, oogonia marker; DDX4, germ cell marker.
[Fig. 18] Histology of a reconstituted ovary at 12 weeks of *in vitro* culture. Compared to the non-addition group (a), in the steroid hormone alone addition group (b) and the retinoic acid (RA) alone administration group (c), a large number of primordial follicles (ZP3/TP63 positive) were produced. In the group (d) in which steroid hormones and RA were added simultaneously, a greater number of primordial follicles were produced. ZP3 and TP63, oocyte markers at ovarian follicle formation stage; DDX4, germ cell marker; DAPI, nucleus.

### DESCRIPTION OF EMBODIMENTS

### 1. Method for producing oocytes or ovarian follicles, or reconstituted ovary containing them

As shown in the Examples below, the inventors have demonstrated that by culturing primate reconstituted ovaries (also referred to as "reaggregated ovaries") under floating culture conditions, reconstituted ovaries having primordial follicle-like structures having properties that are extremely similar to those of primordial follicles *in vivo* can be produced. Therefore, in this specification, unless otherwise specified, (primordial) follicle-like structures are also included in the "(primordial) follicles". An ovarian follicle is a structure containing an oocyte surrounded by granulosa cells (including "pre-granulosa cells"; the same applies to the following), and it is possible to produce oocytes through the above culture. The granulosa cells are somatic cells that surround the oocyte, and flat pre-granulosa cells are cells that differentiate into cuboidal granulosa cells upon activation and express FOXL2. Also, unless otherwise specified, hereinafter, the term "reconstituted ovary" refers to an ovary that is subjected to culture under floating culture conditions (in other words, an ovary as a starting material for culture).

Therefore, the present invention provides a method for producing oocytes or ovarian follicles, or a reconstituted ovary containing oocytes or ovarian follicles, comprising a step of culturing a reconstituted ovary of a primate under a floating culture condition (also referred to as "floating culture") (hereinafter, this may be referred to as the "production method of the present invention"). The oocytes and ovarian follicles obtained using the production method of the present invention can also be isolated and collected from the obtained reconstituted ovary by a method known per se.

In this specification, "floating culture conditions" refer to conditions under which cells or cell aggregates are kept suspended in a culture medium, i.e., conditions under which strong cell-substratum junctions are not formed between cells or cell aggregates and the culture vessel or the extracellular matrix coating the culture vessel, etc.

The medium for floating culture conditions can be prepared by adding medium additives to a basal medium as needed. Examples of the above basal medium include, but are not limited to, RPMI-1640 medium, Eagle's MEM (EMEM), Dulbecco's modified MEM, Glasgow's MEM (GMEM), α-MEM, medium 199, IMDM, DMEM, Hybridoma Serum-Free medium, KnockOut^{™} DMEM (KO DMEM), Advanced^{™} medium (e.g., Advanced MEM, Advanced RPMI, Advanced DMEM/F-12), Chemically Defined Hybridoma Serum-Free medium, Ham's Medium F-12, Ham's Medium F-10, Ham's Medium F12K, DMEM/F-12, ATCC-CRCM30, DM-160, DM-201, BME, Fischer, McCoy's 5A, Leibovitz's L-15, RITC80-7, MCDB105, MCDB107, MCDB131, MCDB153, MCDB201, NCTC109, NCTC135, Waymouth's Medium (e.g., Waymouth's MB752/1), CMRL medium (e.g., CMRL-1066), Williams' medium E, Brinster's BMOC-3 Medium, E8 Medium, StemPro 34, MesenPRO RS (these are from Thermo Fisher Scientific), ReproFF2, Primate ES Cell Medium, ReproStem (these are from Reprocell Inc.), ProculAD (Rohto Pharmaceutical Co., Ltd.), MSCBM-CD, MSCGM-CD (these are from Lonza), EX-CELL302 medium (SAFC) or EX-CELL-CD-CHO (SAFC), ReproMed^{™} iPSC Medium (Reprocell Inc.), Cellartis MSC Xeno-Free Culture Medium (Takara Bio Inc.), TESR-E8 (Veritas Corporation), StemFit (registered trademark) AK02N and AK03N (Ajinomoto Co., Inc.), and mixtures thereof. In particular, Advanced MEM is preferred. The composition of Advanced MEM medium, which is published on the Thermo Fisher Scientific website (https://www.thermofisher.com/jp/ja/home/technical-resources/media-formulation.39.html), is shown in Table 1.

**[Table 1-1]**

| Components | Molecular Weight | Concentration (mg/L) | mM |
|---|---|---|---|
| **Amino Acids** | | | |
| Glycine | 750 | 75 | 0.1 |
| L-Alanine | 89.0 | 8.9 | 0.099999994 |
| L-Arginine hydrochloride | 211.0 | 126.0 | 0.5971564 |
| L-Asparagine | 132.0 | 13.2 | 0.1 |
| L-Aspartic acid | 133.0 | 133 | 0.1 |
| L-Cystine 2HCl | 313.0 | 31.0 | 0.09904154 |
| L-Glulamic Acid | 147.0 | 14.7 | 0.1 |
| L-Histidine hydrochloride-H2O | 210.0 | 42.0 | 0.2 |
| L-Isoleucine | 131.0 | 52.0 | 0.39694658 |
| L-Leucine | 131.0 | 52.0 | 0.39694658 |
| L-Lysine hydrochloride | 183.0 | 72.5 | 0.39617488 |
| L-Methionine | 149.0 | 15.0 | 0.10067114 |
| L-Phenylalanine | 165.0 | 32.0 | 0.19393939 |
| L-Proline | 115.0 | 11 5 | 0.1 |
| L-Serine | 105.0 | 10.5 | 0.1 |
| L-Threanine | 119.0 | 48.0 | 0.40336135 |
| L-Tryptophan | 204.0 | 10.0 | 0.04901961 |
| L-Tyrosine disodium salt dihydrate | 261.0 | 52.0 | 0.19923371 |
| L-Valine | 117.0 | 46.0 | 0.3931624 |

| **Vitamins** | | | |
|---|---|---|---|
| Ascorbic Acid phosphate | 290.0 | 25 | 0.00862069 |
| Choline chloride | 140.0 | 1.0 | 0.007142857 |
| D-Calcium pantothenate | 477.0 | 1.0 | 0.002096436 |
| Folic Acid | 441.0 | 1.0 | 0.0022675737 |
| Niacinamide | 122.0 | 1.0 | 0.008196721 |
| Pyridoxine hydrochloride | 204.0 | 1.0 | 0.004901961 |
| Riboflavin | 376.0 | 0.1 | 2.6595744E-4 |
| Thiamine hydrochloride | 337.0 | 1.0 | 0.002967359 |
| i-Inositol | 180.0 | 2.0 | 0.011111111 |

**[Table 1-2]**

| **Inorganic Salts** | | | |
|---|---|---|---|
| Calcium Chloride (CaCl2) (anhyd.) | 111.0 | 200.0 | 1.8018018 |
| Magnesium Sulfate (MgSO4) (anhyd.) | 120.0 | 97.67 | 0 8139166 |
| Potassium Chloride (KCl) | 75.0 | 400.0 | 5.3333335 |
| Sodium Bicarbonate (NaHCO3) | 84.0 | 2200.0 | 26 190475 |
| Sodium Chloride (NaCl) | 58.0 | 6800.0 | 117.24138 |
| Sodium Phosphate dibasic (Na2HPO4-H2O) | 138.0 | 140.0 | 1.0144928 |

| **Proteins** | | | |
|---|---|---|---|
| AlbuMAX^{®} II | | 400.0 | Infinity |
| Human Transferrin (Holo) | | 7.5 | Infinity |
| Insulin Recombinant Full Chain | 5964.0 | 10.0 | 0.0016767271 |
| Trace Elements | | | |
| Ammonium Metavanadate | 117.0 | 3.0E-4 | 2.5641027E-6 |
| Cupric Sulfate | 250.0 | 0.00125 | 5.0E-6 |
| Manganous Chloride | 198.0 | 5.0 | 0.025252525 |
| Sodium Selenite | 173.0 | 0.005 | 2.8901733E-5 |

| **Other Components** | | | |
|---|---|---|---|
| D-Glucose (Dextrose) | 180.0 | 1000.0 | 5.5555553 |
| Ethanolamine | 98.0 | 19 | 0.019387756 |
| Glutathione (reduced) | 307.0 | 1.0 | 0.0032573289 |
| Phenol Red | 37604.0 | 10.0 | 2.6592915E-4 |
| Sodium Pyruvate | 110.0 | 110 0 | 1.0 |

Furthermore, physiologically active substances, nutritional factors, and the like necessary for cell survival or proliferation can be added to the medium as needed. These medium additives may be added to a medium in advance or may be added during cell culture. During culture, a medium additive may be added in any form, such as one solution or a mixed solution of two or more types of solutions, and an additive may be added continuously or intermittently.

Examples of the physiologically active substances include insulin, IGF-1, transferrin, albumin, coenzyme Q10, various cytokines (interleukins (IL-2, IL-7, IL-15, and the like), stem cell factors (SCFs), activin, etc.), various hormones, and various growth factors (leukemia inhibitory factor (LIF), and basic fibroblast growth factor (bFGF), TGF-β, etc.). Examples of the nutritional factors include sugars, amino acids, vitamins, pyruvates (e.g., sodium pyruvate), hydrolysates, and lipids. Examples of the sugars include glucose, mannose, and fructose, and these sugars may be used alone or in combination of two or more. Examples of the amino acids include L-alanine, L-arginine, L-asparagine, L-aspartic acid, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, L-valine, and non-essential amino acids (NEAAs), and these amino acids may be used alone or in combination of two or more. Examples of the vitamins include ascorbic acid, retinol, retinoic acid, niacin, biotin, pyridoxine, vitamin B12, D-pantothenic acid, choline, folic acid, myo-inositol, niacinamide, pyridoxal, riboflavin, thiamine, cyanocobalamin, and DL-α-tocopherol, and these vitamins may be used alone or in combination of two or more. Examples of the hydrolysates include products obtained by hydrolyzing soybeans, wheat, rice, peas, corn, cottonseed, yeast extracts, and the like. Examples of the lipids include cholesterol, linoleic acid, and linolenic acid.

Further, antibiotics such as kanamycin, streptomycin, penicillin, or hygromycin may be added to the medium as needed. When an acidic substance such as sialic acid is added to the medium, it is desired to adjust the pH of the medium to a neutral range suitable for cell growth, i.e., pH 5 to 9, preferably pH 6 to 8.

The medium used for floating culture may be a serum (e.g., fetal bovine serum (FBS), human serum, horse serum)-containing medium or a serum-free medium. FBS is preferable as serum. From the viewpoint of preventing contamination with components derived from different animal species, it is preferable that the medium does not contain serum or serum derived from the same animal species as the cells to be cultured is used. Here, serum-free medium refers to medium that does not contain unadjusted or unpurified serum. The serum-free medium may contain purified blood-derived components or animal tissue-derived components (e.g., growth factors).

The medium used for floating culture may or may not contain serum substitutes, as well as serum. Examples of serum substitutes include albumin, albumin substitutes such as lipid-rich albumin and recombinant albumin, plant starch, dextrans, protein hydrolysates, transferrin, other iron transporters, fatty acids, insulin, collagen precursors, trace elements, 2-mercaptoethanol, 3'-thioglycerol, and equivalents thereof. Specific examples of serum substitutes include serum substitutes prepared using the method described in WO 98/30679, and commercially available knockout Serum Replacement (KSR) (Life Technologies Corporation), Chemically-defined Lipid concentrated (Life Technologies Corporation), and L-alanine-L-glutamine dipeptide (e.g., Glutamax (Life Technologies Corporation)), etc. Examples of biologically derived factors include platelet-rich plasma (PRP) and culture supernatant components of human mesenchymal stem cells.

Also, the medium preferably contains one or more vitamins. Suitable vitamins include ascorbic acid, retinoic acid, and the like. The term "ascorbic acid" typically means (R)-3,4-dihydroxy-5-((S)-1,2-dihydroxyethyl)furan-2(5H)-one (Cas No: 50-81-7), but also includes "ascorbates" and "ascorbic acid precursors". The term "retinoic acid" typically means all-trans-3,7-dimethyl-9-(2,6,6-trimethyl-1-cyclohexen-1-yl)-2,4,6,8-nonatetraenoic acid (Cas No: 302-79-4), but also includes "retinoic acid salts" and "retinoic acid precursors". Examples of the ascorbates for use in the present invention include, but are not limited to, sodium ascorbate, potassium ascorbate, and calcium ascorbate. Examples of the ascorbic acid precursors for use in the present invention include, but are not limited to, trisodium ascorbyl phosphate, magnesium ascorbyl phosphate (VC-PMg), ascorbyl tetra-hexyldecanoate (VC-IP), and trisodium ascorbyl palmitate phosphate. Examples of the retinoic acid salts for use in the present invention include, but are not limited to, sodium retinoic acid, potassium retinoic acid, and calcium retinoic acid. Examples of the retinoic acid precursors for use in the present invention include, but are not limited to, β-carotene, retinol esters, retinol, and retinal.

The medium preferably also contains one or more hormones. Examples of suitable hormones include steroid hormones. Specific examples of the steroid hormones include progesterone, cortisol, dehydroepiandrosterone, and dehydroepiandrosterone sulfate. Further, vitamins and hormones can be replaced with artificial or natural compounds that have the equivalent steroid structure.

In one embodiment, the floating culture is a culture in a medium containing L-glutamine, ascorbic acid, and 2-mercaptoethanol. As shown in Examples below, addition of retinoic acid to the medium had the effect of facilitating differentiation of oogonia into oocytes, and the effect of facilitating the initiation and progression of meiosis. Also, addition of steroid hormones to the medium facilitated differentiation and maturation of oocytes. Furthermore, it has also been shown that retinoic acid and steroid hormones synergistically increase the efficiency of primordial follicle production. Therefore, preferably, the medium further contains retinoic acid. The medium further contains one or more (preferably all) selected from the group consisting of progesterone, cortisol, dehydroepiandrosterone, and dehydroepiandrosterone sulfate. Further preferably, the medium contains retinoic acid and one or more (preferably all) selected from the group consisting of progesterone, cortisol, dehydroepiandrosterone, and dehydroepiandrosterone sulfate. In this specification, these additives also include those that are produced by decomposition or the like in the medium due to cell secretions or the like (for example, L-alanine-L-glutamine dipeptide from which L-glutamine is produced). Also, in another embodiment, the medium may contain serum.

The concentration of L-glutamine in the medium is not particularly limited, and is, for example, 10 µM to 1 M, preferably 100 µM to 10 mM, and more preferably 1 mM to 3 mM (2 mM in one embodiment). The concentration of ascorbic acid in the medium is not particularly limited, and is, for example, 1 µM to 1 M, preferably 10 µM to 500 µM, and more preferably 100 µM to 200 µM (150 µM in one embodiment). The concentration of 2-mercaptoethanol in the medium is not particularly limited, and is, for example, 0.1 µM to 1 M, preferably 1 µM to 500 µM, and more preferably 10 µM to 200 µM (55 µM in one embodiment). The concentration of retinoic acid in the medium is not particularly limited, and is, for example, 0.1 nM to 5 µM, preferably 1 nM to 1 µM, and more preferably 10 nM to 300 nM (in particular, 10 nM). The concentration of retinoic acid in the medium may be 10 nM to 1 µM. The concentration of progesterone in the medium is 0.01 µg/mL to 10 µg/mL, preferably 0.05 µg/mL to 1 µg/mL, and more preferably 0.1 µg/mL to 0.2 µg/mL (in particular, 0.2 µg/mL). The concentration of cortisol in the medium is 0.01 µg/mL to 10 µg/mL, preferably 0.05 µg/mL to 1 µg/mL, and more preferably 0.1 µg/mL to 0.2 µg/mL (in particular, 0.1 µg/mL). The concentration of dehydroepiandrosterone in the medium is not particularly limited, and is, for example, 0.1 nM to 5 µM, preferably 1 nM to 1 µM, and more preferably 20 nM to 100 nM (in particular, 50 nM). The concentration of dehydroepiandrosterone sulfate in the medium is not particularly limited, and is, for example, 0.1 nM to 5 µM, 1 nM to 1 µM, or 1 nM to 10 nM. Alternatively, the concentration of dehydroepiandrosterone sulfate in the medium is 0.1 µM to 5 mM, preferably 1 µM to 1 mM, and more preferably 1 µM to 100 µM (in particular, 10 µM). The concentration (v/v) of serum such as FBS in the medium is not particularly limited, and is, for example, 0.01% to 50%, preferably 0.01% to 20%, and more preferably 0.01% to 10% (10% in one embodiment). These may be added to the medium at the same time or may be added to the medium at different times.

The culture vessel used for carrying out the floating culture is not particularly limited as long as it is capable of "floating culture", and a person skilled in the art can determine the culture vessel as appropriate. These culture vessels are preferably low cell-adhesive or non-cell-adhesive in order to enable floating culture. It is possible to use, as a culture vessel with low cell-adhesion or non-cell adhesion, a culture vessel whose surface has not been artificially treated (e.g., coated with an extracellular matrix or the like) for the purpose of improving adhesion to cells. Also, the surface of the vessel may be subjected to a treatment for inhibiting adsorption of the extracellular matrix (e.g., Nunclon Sphera treatment, etc.), or may not be subjected to such a treatment. The vessel may be a vessel having a flat bottom, a vessel having a round bottom (e.g., a U-bottom), or a vessel with another shape. Examples of such culture vessels include flasks, tissue culture flasks, dishes, Petri dishes, tissue culture dishes, multi-dishes, microplates, microwell plates, micropores, multi-plates, multi-well plates, chamber slides, Schale, tubes, trays, culture bags, and roller bottles. Further, a bioreactor is an example of a vessel for floating culture.

Culture conditions such as temperature, carbon dioxide concentration, oxygen concentration, and pH can be determined as appropriate based on techniques conventionally used for culturing animal cells. For example, the culture temperature is not particularly limited, and may be 30°C to 40°C, and preferably 37°C. The carbon dioxide concentration may be 1% to 10%, and preferably 2% to 5%. The oxygen concentration may be 1% to 40%, and preferably 1% to 20%.

Mechanical stress plays an important role in maintaining the dormant state of oocytes in mouse primordial follicles, and it has been reported that oocytes can be induced into a dormant state by culturing the oocytes *in vitro* under pressurized conditions (Nagamatsu G, et al., Sci Adv. 5(6): eaav9960 (2019)). Thus, the culture is typically carried out under 1 atm, but at least a part of the culture period may be carried out under reduced pressure or pressurized conditions (e.g., 0 atm to 10 atm, preferably 0 atm to 5 atm, and more preferably 1 atm to 2 atm). For example, a commercially available gas pressure cell stimulator (e.g., AGP series manufactured by Strex Inc.) can be used for the culture under reduced pressure or pressurized conditions.

Since the reconstituted ovary can be maintained for a long period of time using the culture method of the production method of the present invention, the culture period is not particularly limited as long as desired oocytes or ovarian follicles can be obtained, and is selected as appropriate according to the type of primate from which cells are derived.
Typically, the culture period is 9 weeks or more (e.g., 10 weeks or more, 11 weeks or more, 12 weeks or more, 13 weeks or more, or 14 weeks or more). The culture period may be 7 weeks or more (e.g., 8 weeks or more or 9 weeks or more). The upper limit of the culture period is not particularly limited, and is typically 24 weeks or less (e.g., 23 weeks or less, 22 weeks or less, 21 weeks or less, 20 weeks or less, or 19 weeks or less).

In this specification, the term "reconstituted ovary" refers to a structure constructed by aggregating germ cells and fetal ovarian somatic cells, and may include cells other than germ cells and fetal ovarian somatic cells, and may also include multiple types of germ cells or multiple types of fetal ovarian somatic cells. Although the "reconstituted ovary" obtained using the production method of the present invention is derived from the above-mentioned structure, the types of cells that constitute the reconstituted ovary may differ from each other through floating culture.

The reconstituted ovary can be constructed typically by carrying out floating culture (hereinafter, may be referred to as "preculture") for a specific number of days in a medium containing germ cells and fetal ovarian somatic cells (e.g., 1 to 3 days, preferably 2 days).
Thus, the production method of the present invention may include a step of preparing a reconstituted ovary, or a step of obtaining a reconstituted ovary by preculturing germ cells and fetal ovarian somatic cells. Such floating culture can be carried out in the same manner as the floating culture of the production method of the present invention, and all the above description can be applied to the types of basal media, medium additives, culture vessels, culture conditions, and the like used.

In one embodiment, the preculture is a culture in a medium containing one or more (preferably all) selected from the group consisting of L-glutamine, sodium pyruvate, 2-mercaptoethanol, and a ROCK inhibitor. Also, in another embodiment, the medium may contain serum. In particular, it is preferable that a ROCK inhibitor is contained in the preculture from the viewpoint of facilitating cell aggregation. Examples of such ROCK inhibitors include Y-27632, Fasudil/HA1077, H-1152, and Wf-536. When Y-27632 is used as a ROCK inhibitor, the concentration of Y-27632 in the medium in the preculture is not particularly limited, and is, for example, 0.1 µM to 1 mM, preferably 1 µM to 100 µM, and more preferably 5 µM to 20 µM (10 µM in one embodiment).

In the preculture, the concentration of L-glutamine in the medium is not particularly limited, and is, for example, 10 µM to 1 M, preferably 100 µM to 10 mM, and more preferably 1 mM to 3 mM (2 mM in one embodiment). The concentration of sodium pyruvate in the medium is not particularly limited, and is, for example, 10 µM to 100 mM, preferably 100 µM to 10 mM, and more preferably 500 µM to 5 mM (1 mM in one embodiment). The concentration of 2-mercaptoethanol in the medium is not particularly limited, and is, for example, 0.1 µM to 1 M, preferably 1 µM to 500 µM, and more preferably 10 µM to 200 µM (100 µM in one embodiment). The concentration (v/v) of serum such as FBS in the medium is not particularly limited, and is, for example, 0.01% to 50%, preferably 0.01% to 20%, and more preferably 0.01% to 10% (10% in one embodiment). These may be added to the medium simultaneously or may be added to the medium at different times.

In this specification, the term "germ cells" refers to cells expressing at least any of DDX4 and DAZL, and preferably cells expressing at least DDX4. Examples of the germ cells used in the present invention include oogonia, primordial germ cells (PGCs), and primordial germ cell-like cells (PGCLCs). The "oogonia" are cells at the stage when primordial germ cells proliferate in the ovary through repeated cell division, and refer to cells expressing at least one of NANOG, POU5F1, TFAP2C, and PDPN. The term "primordial germ cells" refers to cells that have the capacity of differentiating into oogonia and spermatogonia, and that express at least any of INTEGRINα6, EpCAM, BLIMP 1, and TFAP2C. The term "primordial germ cell-like cells" refers to cells having properties equivalent to PGCs, which are produced through inducing differentiation from pluripotent stem cells.

Also, the term "fetal ovarian somatic cells" in this specification refers to cells other than germ cells contained in the fetal ovary. The fetal ovaries may be fetal ovaries at any weeks as long as they contain fetal ovarian somatic cells, and may vary depending on the species of primate from which the cells are derived; however, fetal ovaries at 6 to 18 weeks post-fertilization (6 to 18 wpf) are preferred.

Specifically, germ cells and fetal ovarian somatic cells can be prepared, for example, by surgically collecting ovaries from a living body and dissociating the germ cells and somatic cells that constitute the ovaries into single cells through enzymatic treatment (e.g., trypsin, etc.). In this case, germ cells (fetal ovarian germ cells) present in the ovaries derived from the living body can be isolated through cell sorting such as FACS (fluorescence-activated cell sorting) and MACS (Magnetic activated cell sorting) using a germ cell marker (e.g., PGC-specific marker INTEGRINα6, EpCAM, or the like) as an indicator, thereby isolating fetal ovarian germ cells and fetal ovarian somatic cells. Alternatively, fetal ovarian germ cells and fetal germ somatic cells may not be dissociated and may be used as a cell population containing these cells. These cells or cell populations can also be cryopreserved. Note that, in this specification, unless otherwise specified, the term "cells" include a "cell population". The cell population may be constituted by one type of cells, or may be constituted by two or more types of cells.

The germ cells and fetal ovarian somatic cells may be also prepared by inducing differentiation from pluripotent stem cells. The term "pluripotent stem cells" refers to stem cells that can differentiate into tissue or cells having various different forms or functions in the body, and that can also differentiate into cells of any lineages of the three germ layers (endoderm, mesoderm and ectoderm). Examples of pluripotent stem cells to be used for the present invention include induced pluripotent stem cells (iPS cells), embryonic stem cells (ES cells), embryonic stem cells from cloned embryos obtained by nuclear transfer (nuclear transfer Embryonic stem cells; ntES cells), multipotent germline stem cells (mGS cells) and embryonic germ cells (EG cells), of which iPS cells (and more preferably human iPS cells) are preferred. When the pluripotent stem cells are ES cells or arbitrary cells derived from human embryos, the cells may be obtained by destruction of the embryo or they may be obtained without destruction of the embryo, but from an ethical point of view, preferably they are cells obtained without destruction of the embryo.

ES cells are stem cells having pluripotency and auto-replicating proliferation potency, established from the inner cell mass of an early embryo (such as the blastocyst) of a mammal such as a human or mouse. ES cells were discovered in mice in 1981 (M.J. Evans and M.H. Kaufman (1981), Nature 292:154-156), and ES cell lines were later established in primates including humans and monkeys (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165). ES cells can be established by extracting the inner cell mass from the blastocyst of a fertilized egg of a target animal and culturing the inner cell mass on a fibroblast feeder. Alternatively, ES cells can be established using a single embryo blastomere alone during the cleavage stage before the blastocyst stage (Chung Y. et al. (2008), Cell Stem Cell 2: 113-117), or they can be established using a developmentally arrested embryo (Zhang X. et al. (2006), Stem Cells 24: 2669-2676.).

ntES cells are ES cells derived from a clone embryo prepared by nuclear transfer, and they have approximately the same properties as fertilized egg-derived ES cells (Wakayama T. et al. (2001), Science, 292:740-743; S. Wakayama et al. (2005), Biol. Reprod., 72:932-936; Byrne J. et al. (2007), Nature, 450:497-502). In other words, ntES (nuclear transfer ES) cells are ES cells established from the inner cell mass of a cloned embryo-derived blastocyst obtained by exchanging an unfertilized egg nucleus with a somatic cell nucleus. Combinations of nuclear transfer (Cibelli J.B. et al. (1998), Nature Biotechnol., 16:642-646) and ES cell preparation techniques (described above) are used to prepare ntES cells (Wakayama, S. (2008), Jikken Igaku, Vol.26, No.5 (special edition), pp.47-52). For nuclear transfer, a somatic cell nucleus may be implanted into a mammalian enucleated unfertilized egg and cultured for several hours for reprogramming.

The ES cell line used for the present invention may be human ES cell lines, the different human ES cell lines established by the University of Wisconsin, NIH, Riken, Kyoto University, the National Center for Child Health and Development, Cellartis, etc., for example, may be used. Specific examples of human ES cell lines include CHB-1 to CHB-12 lines, RUES1 line, RUES2 line and HUES1 to HUES28 lines, etc., distributed by ESI Bio Co., H1 and H9 lines, etc., distributed by WiCell Research, KhES-1 line, KhES-2 line, KhES-3 line, KhES-4 line, KhES-5 line, SSES1 line, SSES2 line and SSES3 lines, etc., distributed by Riken.

iPS cells are cells obtained by reprogramming of mammalian somatic cells or undifferentiated stem cells by introduction of specific factors (nuclear reprogramming factors). A large number of iPS cells currently exist, among which there may be used human iPSCs established by introduction of the 4 factors Oct3/4·Sox2·Klf4·c-Myc into human fibroblasts by Yamanaka (Takahashi K, Yamanaka S., et al. Cell, (2007) 131:861-872.), Nanog-iPSCs established by selecting of Nanog expression markers after introduction of the 4 factors (Okita, K., Ichisaka, T. and Yamanaka, S. (2007). Nature 448, 313-317.), iPSCs produced by methods without c-Myc (Nakagawa M, Yamanaka S., et al. Nature Biotechnology, (2008) 26, 101-106), iPSCs established by introduction of 6 factors by a virus-free method (Okita K et al. Nat. Methods 2011 May; 8(5):409-12, Okita K et al. Stem Cells. 31(3):458-66.), and so on. The induced pluripotent stem cells established by introduction of the 4 factors OCT3/4·SOX2·NANOG·LIN28, created by Thomson et al. (Yu J., Thomson JA. et al., Science (2007) 318: 1917-1920), the induced pluripotent stem cells created by Daley et al. (Park IH, Daley GQ. et al., Nature (2007) 451:141-146) and the induced pluripotent stem cells created by Sakurata et al. (Japanese Unexamined Patent Publication No. 2008-307007), etc., may also be used.

In addition, any induced pluripotent stem cells publicly known in the field as described in published journal (for example, Shi Y., Ding S., et al., Cell Stem Cell, (2008) Vol.3, Issue 5, 568-574, Kim JB., Scholer HR., et al., Nature, (2008) 454, 646-650; Huangfu D., Melton, DA., et al., Nature Biotechnology, (2008) 26, No 7, 795-797), or patent publications (for example, Japanese Unexamined Patent Publication No. 2008-307007, Japanese Unexamined Patent Publication No. 2008-283972, US2008-2336610, US2009-047263, WO2007-069666, WO2008-118220, WO2008-124133, WO2008-151058, WO2009-006930, WO2009-006997 and WO2009-007852), may also be used.

Induced pluripotent stem cell lines that are iPSC lines established by NIH, Riken, Kyoto University, etc., for example, may be used as well. Examples include human iPSC lines such as HiPS-RIKEN-1A line, HiPS-RIKEN-2A line, HiPS-RIKEN-12A line, Nips-B2 line, etc., by Riken, 253G1 line, 253G4 line, 1201C1 line, 1205D1 line, 1210B2 line, 1383D2 line, 1383D6 line, 201B7 line, 409B2 line, 454E2 line, 606A1 line, 610B1 line, 648A1 line, 1231A3 line, FfI-01s04 line, etc., by Kyoto University, with the 1231A3 line being preferred.

mGS cells are pluripotent stem cells derived from the testis and are the source cells for spermatogenesis. Like ES cells, these cells can be induced to differentiate into cells of various lineages, and have properties such as the ability to produce chimeric mice when transplanted into mouse blastocysts (Kanatsu-Shinohara M. et al. (2003) Biol. Reprod., 69:612-616; Shinohara K. et al. (2004), Cell, 119:1001-1012). These cells are capable of auto-replication in a culture medium containing glial cell line-derived neurotrophic factor (GDNF), and by repeated passage under the same culture conditions as those for ES cells, germline stem cells can be obtained (Takebayashi, M. et al. (2008), Jikken Igaku, Vol.26, No.5 (special edition), pp.41-46, Yodosha Co., Ltd. (Tokyo, Japan)).

EG cells are established from the primordial germ cells in the embryonic period and have pluripotency similar to that of ES cells. They can be established by culturing primordial germ cells in the presence of substances such as LIF, bFGF, and stem cell factor (Matsui Y. et al. (1992), Cell, 70:841-847; J. L. Resnick et al. (1992), Nature, 359:550-551).

Differentiation of pluripotent stem cells into PGCLCs can be induced by a method known per se. Specifically, induction of PGCLCs from induced pluripotent stem cells can be carried out using the method described in Hayashi K, et al., Cell 2011 Aug 19;146(4):519-32. Alternatively, the methods described in WO 2017/002888 and WO 2022/039279 may be used, specifically, (1) pluripotent stem cells are cultured in a culture medium containing activin A and a GSK-3β inhibitor to induce early mesoderm-like cells (iMeLCs), and (2) the iMeLCs are cultured in the presence of BMP, thereby inducing differentiation into PGCLCs.

Basal media for inducing differentiation of pluripotent stem cells into PGCLCs include, but are not limited to, the basal media mentioned as examples for use in the above-mentioned floating culture. The basal media may contain other medium additives known per se, such as those mentioned as examples for use in the above-mentioned floating culture.

The medium may be a serum-containing medium or a serum-free medium (SFM). Preferably, a serum-free medium is used. The concentration (v/v) of serum (e.g., FBS or the like) in the medium may be, for example, 20% or less, 5% or less, 2% or less, or 0% (i.e., serum-free). The SFM may or may not include any serum substitutes such as KSR.

The concentration of activin A in the medium for inducing differentiation of pluripotent stem cells into iMeLCs is, for example, 5 to 100 ng/ml, 10 to 90 ng/ml, 15 to 80 ng/ml, 20 to 70 ng/ml, or 30 to 60 ng/ml. Preferably, the concentration of activin A in the medium is 50 ng/ml. Examples of GSK-3β inhibitors used when iMeLCs are induced from pluripotent stem cells include LiCl, BIO, SB216763, GSK-3β inhibitors VII, L803-mts, and CHIR99021, and CHIR99021 is preferred. The concentration of CHIR99021 in the medium is not particularly limited, and is preferably 0.1 µM to 50 µM. The pluripotent stem cells can be induced to differentiate into iMeLCs by culturing the pluripotent stem cells at a seeding density of 10⁴ to 10⁵ cells/cm², preferably 2 to 6×10⁴ cells/cm², at 30°C to 40°C for less than 60 hours, preferably 42 hours.

Examples of BMPs used to induce differentiation of iMeLCs into PGCLCs include BMP2, BMP4, and BMP7, and BMP4 is preferred. The concentration of BMP in the medium is, for example, 50 to 100 ng/ml, 100 to 800 ng/ml, or 150 to 600 ng/ml. Preferably, the concentration of BMP in the medium is 200 ng/ml. iMeLCs can be induced to differentiate into PGCLCs by culturing iMeLCs at a seeding density of 1 to 50×10³ cells/cm², preferably 5 to 20×10³ cells/cm², at 30°C to 40°C for 4 to 10 days, preferably 5 to 8 days, more preferably 6 days.

It is also possible to induce differentiation of PGCs or PGCLCs into oogonia, and such differentiation can be carried out, for example, by the method described in Non Patent Literature 3. Specifically, mouse fetal somatic cells are isolated from the ovary of a mouse fetus, and then the cells are reaggregated with PGCs or PGCLCs to construct a heterologous reconstituted ovary. Oogonia can then be produced by culturing the ovary for a specific number of days (e.g., 77 days or more) using the air-liquid interface culture method.

Differentiation of pluripotent stem cells into fetal ovarian somatic cells can be induced according to the method of Hayashi et al. (Yoshino Y, et al., Science, 373(6552): eabe0237 (2021)), for example. Fetal ovarian somatic cell-like cells (FOLSCs), which have properties similar to those of fetal ovarian somatic cells, can be induced through sequential differentiation of pluripotent stem cells into nascent mesoderm, intermediate mesoderm, and coelomic epithelium states. Specifically, in the case of mice, the following method is exemplified. The pluripotent stem cells differentiate into EpiLCs using activin A and bFGF. EpiLCs are cultured in low cell adhesion U-Shaped Bottom 96-well plates containing 14 µM CHIR99021, 1 ng/ml BMP4, and 50 ng/ml epidermal growth factor (EGF) for 2 days, followed by culture for an additional 2 days with 3 µM retinoic acid, 30 ng/ml sonic hedgehog, 1 µM PD0325901, 50 ng/ml EGF, and 1 ng/ml BMP4. Then, the cells are cultured with 20 ng/ml BMP4 and 2 ng/ml FGF9 for additional 1 or 2 days.

As shown in Examples below, primordial follicles having primary oocytes that have completed at least meiotic prophase I can be obtained using the production method of the present invention. Also, by culturing for an even longer period of time, the oocytes may mature into secondary oocytes, and the ovarian follicles may also mature from the primordial follicles into primary, secondary, and mature ovarian follicles. Thus, the oocytes obtained using the production method of the present invention may be either primary oocytes (including cells in the process of maturing into secondary oocytes) or secondary oocytes. The ovarian follicles obtained using the production method of the present invention may be any of primordial follicles (including ovarian follicles in the process of maturing into primary ovarian follicles), primary ovarian follicles (including ovarian follicles in the process of maturing into secondary ovarian follicles), secondary ovarian follicles (including ovarian follicles in the process of maturing into mature follicles), and mature follicles.

Also, the oocytes obtained using the production method of the present invention may be further matured using a method known per se. Therefore, in another embodiment of the present invention, provided is a method for producing an ovum (may be referred to as the "ovum production method of the present invention" hereinafter), comprising a step of maturing the oocyte obtained using the production method of the present invention. Examples of such a method include the method of Telfer et al. (McLaughlin M, et al., Mol Hum Reprod, 24(3):135-142 (2018)). Specifically, an ovum can be produced by (1) culturing the reconstituted ovaries in a serum-free medium for a specific number of days (e.g., 8 days), (2) isolating secondary/multilayered follicles from the reconstituted ovaries and individually culturing the isolated ovarian follicles in the serum-free medium in the presence of activin A, (3) after a specific number of days (e.g., 8 days), collecting cumulus-oocyte complexes (COCs) by applying moderate pressure to the cultured ovarian follicles, and (4) culturing the collected complexes on a membrane in the presence of activin A and FSH for an additional specific number of days (e.g., 4 days).

Alternatively, the step of maturing oocytes can be carried out by transplanting oocytes or ovarian follicles obtained by the production method of the present invention, or a reconstituted ovary containing these oocytes or ovarian follicles, beneath the kidney capsule or ovarian capsule of a primate. A method known per se can be used as the transplantation method.

In this specification, the term "primate" refers to a mammalian animal belonging to the order Primates. The origin of the above-mentioned induced pluripotent stem cells, germ cells, and fetal ovarian somatic cells is not particularly limited, as long as they are from primates. Primates may be strepsirrhines or haplorhines. Examples of strepsirrhines include lemurs, aye-ayes, and lorises. Examples of haplorhines include tarsiers, cercopitheciids (e.g., cynomolgus monkeys, rhesus monkeys, Japanese macaques, Formosan rock macaques, and the like), gibbons, humans, spider monkeys, capuchin monkeys, and Saki monkeys. In particular, cercopitheciids such as cynomolgus monkeys, and humans are preferred, and humans are more preferred.

In another embodiment, provided is a method for maintaining a reconstituted ovary, comprising a step of culturing a reconstituted ovary of a primate under a floating culture condition. All the above descriptions regarding the production method of the present invention can be applied to a specific culture method and the like of this method. Although the wording "maintaining the reconstituted ovary" means that the morphology of the reconstituted ovary is maintained without collapse, and the types of cells that constitute the reconstituted ovary may differ from each other through floating culture. In yet another embodiment, provided are oocytes and ovarian follicles, which are obtained (or obtainable) by the production method of the present invention, or a reconstituted ovary that contains these oocytes and ovarian follicles, and an ovum obtained (or obtainable) by the ovum production method of the present invention. Preferably, the above cells are provided in the state of being cryopreserved using a method known per se. For example, the above oocytes and ovarian follicles are not only the starting materials for producing an ovum, but are also extremely useful for understanding the mechanism of germ cell development.

### 2. Kit for producing oocytes or ovarian follicles, or reconstituted ovary containing them

The present invention further provides a kit for producing oocytes or ovarian follicles, or a reconstituted ovary containing oocytes or ovarian follicles (may be referred to as the "production kit of the present invention" hereinafter), comprising a culture vessel for floating culture, L-glutamine, ascorbic acid, and 2-mercaptoethanol. The production kit of the present invention may comprise serum. The production kit of the present invention may comprise retinoic acid and/or one or more (preferably all) molecules of selected from the group consisting of progesterone, cortisol, dehydroepiandrosterone, and dehydroepiandrosterone sulfate.

The production kit of the present invention may comprise a basal medium and/or medium additives such as a physiologically active substance. All the contents described in the above section "1. Method for Producing Oocytes or Ovarian Follicles, or Reconstituted Ovary Containing Them" are applied to the definitions, specific examples, and the like of the culture vessel for floating culture, serum, L-glutamine, ascorbic acid, 2-mercaptoethanol, retinoic acid, progesterone, cortisol, dehydroepiandrosterone, dehydroepiandrosterone sulfate, the basal media, and medium additives, which are included in the production kit of the present invention.

The present invention will be described more specifically with reference to the following examples. It is to be noted, however, that the present invention is not limited to these examples by any means.

### EXAMPLES

### Example 1: cy fetal oocyte development

cy PGCs are specified in the nascent amnion from around embryonic day (E) 11, migrate through the developing hindgut endoderm and mesentery, and colonize the incipient embryonic gonads from around E30 (Sasaki K, et al., Dev Cell, 39: 169-185 (2016)). In female embryos, embryonic gonads increase their size progressively and migrates into ovaries by around E40, whereas cyPGCs continue to colonize the developing gonads and also increase their numbers mitotically as oogonia (Sasaki K, et al., Dev Cell, 39: 169-185 (2016), Sasaki K, et al., Cell reports, 35: 109075 (2021)). Thereafter, fetal oocyte development ensues in fetal ovaries (Fig. 1A). To create a benchmark for the *in vitro* reconstitution of cy fetal oocyte development, a careful characterization of cy fetal oocyte development *in vivo* was performed from 8 weeks post-fertilization (8 wpf: at E56-58) to 18 wpf (at E127 and 128) at two-week intervals (Fig. 1B, Fig. 2A). The gestation period of cynomolgus monkeys is around 22 weeks.

### Histological analysis

At 8 wpf, the ovaries exhibited a relatively uniform histology with abundant oogonia and no clear differentiation of ovarian cortex and medulla (Fig. 1C). Oogonia were large in size (about 12 µm in diameter), bore a round nucleus with pale hematoxylin staining and a prominent eosinophilic nucleolus, and formed anastomosing cord-like structures intercalated by relatively small cells with densely stained nuclei and a large nuclear-cytoplasmic ratio (Fig. 1C). The interstitial stromal cells were evident, showed a spindle shape with a large nuclear-cytoplasmic ratio and were embedded in loose connective tissues presumably synthesized by themselves (Fig. 1C). Immunofluorescence (IF) analyses revealed that all oogonia expressed DDX4, an RNA-binding protein expressed in oogonia and oocytes (Toyooka Y, et al., Mech Dev, 93: 139-49 (2000)), whereas small, intercalated cells expressed FOXL2, a key transcription factor (TF) for granulosa-cell development (Crisponi L, et al., Nat Genet, 27: 159-66 (2001)), demonstrating their granulosa-cell identity (Fig. 2B). At 10 wpf, the ovaries grew in size with the acquisition of a kidney-like shape, developing medulla consisting mainly of interstitial cells (Fig. 1B). The cortex was populated with abundant oogonia, and the outer and inner cortexes did not show overt histological differences (Fig. 1D, Fig. 2C). At 12 wpf, while oogonia were still abundant, germ cells with larger nuclei with condensed, thread-like chromatin became apparent, particularly in the inner cortex (Fig. 1D, Fig. 2C), indicating that oogonia enter into the meiotic prophase I to differentiate into oocytes. At 14 wpf, oocytes at meiotic prophase I became dominant even in the outer cortex, and relatively large oocytes delineated by granulosa cells, i.e., primordial follicles, began to be observed in the inner cortex (Fig. 1D, Fig. 2C). At 16 wpf, primordial follicles were observed in the outer cortex as well, and at 18 wpf, they became dominant both in the outer and inner cortexes (Fig. 1D, Fig. 2C). On the other hand, even at 18 wpf, oogonia-like cells with no apparent sign of meiotic entry were observed in the outer cortex (Fig. 1D, Fig. 2C), demonstrating a substantial asynchrony in cy oocyte development. The (pre-)granulosa : germ-cell ratio (FOXL2-positive(⁺) : DDX4⁺ cell ratio) was measured to find that it was about 2 at 8 wpf, dropped to and remained about 1 at 10 and 12 wpf, and increased sharply after 14 wpf, particularly in the inner cortex, reaching about 6 and about 3 at 18 wpf in the inner and outer cortexes, respectively (Fig. 2D). This finding is consistent with the idea that germ cells increase their number by mitotic expansion of oogonia at least until 10 wpf and then their number decreases dramatically after 14 wpf, presumably due to apoptosis associated with meiotic prophase (see below), whereas granulosa cells maintain their number relatively constantly or proliferate slowly after their specification.

### Key-marker expression

An scRNA-seq analysis classified human female fetal germ cells into four cell types: "mitotic" (oogonia), "retinoic acid (RA)-responsive," "meiotic," and "oogenesis (Hereinafter referred to as "oogenic.")" (Li L, et al., Cell Stem Cell, 20: 858-873 e4 (2017)). Accordingly, the cy fetal oocyte development was evaluated with the expression of key markers of the four cell types. At 8 wpf, a vast majority of DDX4⁺ cells expressed markers for oogonia: nearly all DDX4⁺ cells were positive for NANOG and around 80% were positive for POU5F1, TFAP2C, and PDPN, whereas only a minority (about 10%) in the inner cortex expressed ZGLP1, a determinant for the oogenic fate and a marker for RA-responsive cells (Fig. 1E, Figs. 2E and F) (Li L, et al., Cell Stem Cell, 20: 858-873 e4 (2017), Nagaoka S, I., et al., Science 367 (2020)). At 10 wpf, despite their histological similarity to the cells at 8 wpf, germ cells expressing oogonia markers decreased dramatically, particularly in the inner cortex; on the other hand, those expressing markers for RA-responsive cells increased (ZGLP1: about 40-50%; STRA8: about 10-20%) and some (about 10-20%) expressed SYCP3 (Fig. 1E, Fig. 2F), an axial component of the synaptonemal complex (SC) that initiates characteristic expression from the pre-leptotene stage of meiosis (Yuan L, et al., Science, 296: 1115-8 (2002), Yuan L, et al., Mol Cell 5: 73-83 (2000)) (see below). While the oogonia markers, NANOG, POU5F1, and TFAP2C, showed a uniform distribution across oogonia nuclei, the RA-responsive cell markers, ZGLP1 and STRA8, exhibited a granular distribution (Fig. 1E, Fig. 2E). At 12 wpf, germ cells expressing oogonia markers continued to decrease and those expressing markers for RA-responsive and meiotic cells (SYCP3, DMC1, γH2AX, SYCP1) continued to increase, and at 14 wpf, oogonia became minor (about 5%), meiotic cells became predominant (about 50%), and cells expressing oogenic markers (ZP3 and TP63) appeared in both the outer (about 10%) and inner (about 30%) cortexes (Fig. 1E, Fig. 2F). γH2AX, a marker for double-strand breaks (DSBs) upon meiotic recombination (Fernandez-Capetillo O, et al., J Cell Biol, 163: 15-20 (2003)), exhibited a dotted distribution in meiotic nuclei, while SYCP1, another axial component of SC (Meuwissen RL, et al., EMBO J, 11: 5091-100 (1992)), showed a typical thread-like distribution along synapsed homologous chromosomes in nuclei corresponding to the late zygotene or pachytene stage of the meiotic prophase I (see below). At 16 and 18 wpf, the oogenic cells (oocytes at the diplotene stage in the primordial follicles) (see below) were dominant in the inner cortex and the meiotic cells were abundant in the outer cortex (Fig. 1E, Fig. 2F). In the oogenic cells, ZP3 was localized on the plasma membrane or in the extracellular space between oocytes and surrounding granulosa cells, indicating the formation of an incipient zona pellucida (ZP) (Fig. 1E). TP63, FIGLA, and NOBOX, TFs critical for oogenesis (Liang L, et al., Development, 124: 4939-47 (1997), Suh EK, et al., Nature, 444: 624-8 (2006), Suzumori N, et al., Mech Dev, 111: 137-41 (2002)), all showed a uniform localization in the nucleoplasm, except in the nucleolus, of the oocytes, whereas NLRP5, a maternal effect protein essential for early embryonic development (Tong ZB, et al., Nat Genet, 26: 267-8 (2000)), exhibited punctate localization throughout the cytoplasm (Fig. 1E, Fig. 2E). LAMININ, a marker for the basement membrane, delineated the basal side of the squamous granulosa cells composing primordial follicles, marking both single and sometimes multiple primordial follicles (Fig. 2E).

Next, the expression of Ki67, a marker for cell proliferation (Gerdes, Schwab et al., 1983), and the expressions of cleaved poly ADP-ribose polymerase (cPARP) and CASPASE 3 (cCAS3), markers for apoptosis (Lazebnik YA, et al., Nature, 371: 346-7 (1994), Tewari M, et al., Cell, 81: 801-9 (1995)), were examined. At 8 wpf, a majority of oogonia (about 70%) expressed Ki67, which was localized mainly around the nucleolus (Figs. 2E and F). Although germ cells expressing oogonia markers decreased dramatically after 10 wpf, Ki67⁺ cells were observed throughout the stages examined (about 40%) (Fig. 2F); these cells would have represented RA-responsive and meiotic cells at an early stage of meiosis (see below). Germ cells with apoptotic markers were not detected at 8 wpf, but from 10 to 14 wpf with the onset and progression of meiotic prophase, and at 16 and 18 wpf with the formation of primordial follicles, about 10% and about 15% of germ cells, respectively, were shown to have apoptotic markers (Figs. 2E and F). The apoptotic primordial follicles showed cCAS3, but not cPARP (Fig. 2F).

### Progression of the meiotic prophase I

The progression of meiotic prophase I was determined based on the chromatin architecture visualized by DAPI staining and the expression/localization of SYCP3 and DMC1, the latter being involved in single-strand invasion for homologous recombination (Bishop DK, et al., Cell, 69: 439-56 (1992)) (Fig. 1F): mitotic oogonia did not show overt signs of chromosome condensation, expressing neither SYCP3 nor DMC1. The pre-leptotene oocytes initiated chromosome condensation and expressed SYCP3, which localized around the nucleolus, but did not show DMC1. The leptotene oocytes exhibited further chromosome condensation and expressed SYCP3, which was localized both around the nucleolus and condensing chromosomes, in addition to DMC1, which was localized on condensing chromosomes. The zygotene oocytes exhibited signs of chromosome pairing and expressed SYCP3 and DMC1, which were localized on the paired chromosomes, with DMC1 showing multiple strongly stained foci. The early pachytene oocytes exhibited robust chromosome pairing with a clear axial structure, and expressed SYCP3 and DMC1, which showed co-localization on multiple strongly stained foci, most likely crossover recombination sites of homologous chromosomes. The late pachytene oocytes were similar to the early pachytene oocytes, except that the former no longer expressed DMC1. The diplotene oocytes were larger in size, exhibited signs of diakinesis, and repressed both SYCP3 and DMC1. With this classification, a majority of germ cells were categorized as mitotic until 10 wpf, and it was demonstrated that the first meiotic division initiates in the inner cortex at 10 wpf and gradually proceeds during cy fetal development, with diplotene oocytes that complete the prophase appearing from 14 wpf onwards (Fig. 1G). Collectively, these data update the classical observations in rhesus monkeys *(Macaca Mulatta)* (Baker TG, J Anat 100: 761-76 (1966)) and create a cytological basis for the progression of cy fetal oocyte development, demonstrating that initiation of first meiotic division and overt morphological changes require around 2 weeks from the repression of oogonia markers and that the meiotic prophase I takes 4-6 weeks to complete.

### Example 2: Cy fetal oocyte development in xenotransplanted cy reconstituted ovaries (cy rOvaries)

Next, it was examined whether cy fetal oocyte development proceeds upon xenotransplantation of cy reaggregated ovaries (cy reconstituted ovaries; rOvaries) into immunodeficient mice (Fig. 3A). Cy fetal ovaries were isolated at 8 wpf, dissociated into single cells (about 6.0 x 10⁵ cells per ovary), about 50,000 cells (approximately 5,000 germ cells and 45,000 somatic cells) were reaggregated under a floating condition for two days to generate cy reconstituted ovaries, they were transplanted under the surface epithelium of a kidney of immunodeficient mice, and cy oocyte development was analyzed at 3-week intervals.

At 3-weeks post-transplantation (3 w-ptp), cy reconstituted ovaries bore an integrated structure and reconstructed anastomosing cords intercalated with stromal cells (Fig. 3B, Fig. 2G). A majority of germ cells showed an immature morphology representing oogonia, while some exhibited larger nuclei with condensed chromatin, indicative of meiotic entry (Fig. 3B). Consistent with these findings, about 40% of germ cells expressed oogonia markers (NANOG, POU5F1, TFAP2C, PDPN), while about 50% expressed ZGLP1, about 30% expressed SYCP3, and about 10% expressed meiotic markers (DMC1, γH2AX, SYCP1) (Figs. 3C and D). At 6 w-ptp, oocyte development progressed further in cy reconstituted ovaries, with a majority of germ cells showing the nuclear morphology in the meiotic prophase I, including that of the pachytene stage with synapsed chromosome pairs (<10% positive for oogonia markers, about 60% positive for SYCP3, and about 30% positive for DMC1, γH2AX, SYCP1), but there were no cells expressing the oogenic markers (Figs. 3B-D, Fig. 2G). From 9 w-ptp onwards, there were differentiated primordial follicle-like complexes with an oocyte expressing ZP3 and TP63 surrounded by a single layer of squamous granulosa cells expressing FOXL2, and by 15 w-ptp, such complexes became dominant (Figs. 3B-D). These findings demonstrate that, upon xenotransplantation into mice, cy fetal oocyte development and the meiotic prophase I proceed apparently normally, although the developmental progression seemed somewhat slower compared to that *in vivo.* Cy reconstituted ovaries were examined at 21 w-ptp, and it was found that although they maintained a distinct structure, only granulosa and stromal cells survived, and essentially all oocytes were degenerated (Fig. 3E), indicating that further follicle growth does not proceed under the current xenotransplantation condition.

### Example 3: Reconstitution of cy fetal oocyte development in vitro

Next, it was explored whether an *in vitro* culture would promote appropriate cy fetal oocyte development in cy reconstituted ovaries. An air-liquid interface culture that supports the development of both mouse embryonic ovaries and reconstituted ovaries was first examined (Hikabe O, et al., Nature, 539: 299-303 (2016), Morohaku K, et al., Proc Natl Acad Sci USA, 113: 9021-6 (2016)). Cy reconstituted ovaries with about 50,000 ovarian cells at 8 wpf were generated and cultured on the Transwell-COL membrane insert (Fig. 4A). Initially, cy reconstituted ovaries on the Transwell-COL membrane showed an integrated structure, but unlike in mouse reconstituted ovaries, some cells began to spread out from cy reconstituted ovaries from as early as 1 week of *in vitro* culture (1 w-ivc), and at 3 w-ivc, many cells had migrated out, leaving the cy reconstituted ovaries smaller and flattened (Fig. 4B, Fig. 5A). The cell spreading continued thereafter, and at 6 w-ivc *(in vitro* culture), the cy reconstituted ovaries were much diminished compared to their original sizes (Fig. 4B, Fig. 5A). Histological and IF analyses revealed that at 3 w-ivc, cy reconstituted ovaries bore ovarian cords, but such areas were limited with a small number of germ cells, and at 6 w-ivc, although FOXL2⁺ granulosa cells formed some clusters, there remained only a few germ cells with degenerating morphology (Fig. 4B). It was examined whether cy reconstituted ovaries can be maintained on a culture membrane coated with various extracellular matrix components, including collagen (type I, III, IV, V, VI), fibronectin, laminins, and their combinations (Fig. 5A). Under some conditions, such as on the membrane coated with collagen type I, III, IV, V, and VI, or iMatrix (laminin 511), cy reconstituted ovaries maintained their integrity up to 5 w-ivc, but thereafter, they began to collapse, i.e., some cells migrated out, and under all conditions examined, cy reconstituted ovaries were flattened out by 12 w-ivc (Fig. 5A). These findings indicate that cy reconstituted ovaries require a condition distinct from that of mouse reconstituted ovaries for appropriate *in vitro* development.

It was therefore explored whether cy reconstituted ovaries can be maintained for the long-term under a floating condition (Fig. 4A). When grown by floating culture in αMEM containing 10% fetal bovine serum (FBS), a basal condition for mouse reconstituted ovary culture (Hikabe O, et al., Nature, 539: 299-303 (2016), Morohaku K, et al., Proc Natl Acad Sci USA, 113: 9021-6 (2016)), at 6 w-ivc, cy reconstituted ovaries showed an integrated appearance outlined by a few layers of squamous epithelial cells, although their central areas appeared somewhat necrotic, and a relatively large number of DDX4⁺ germ cells survived with intercalating FOXL2⁺ granulosa cells (Fig. 5B). Thirteen different basal media, including αMEM (αMEM, GMEM, DMEM, KO DMEM, Advanced MEM, DMEM/F-12, Waymouth's, McCoy's 5A, CMRL, IMDM, Advanced RPMI, StemPro 34, MesenPRO RS), were examined, and it was found that Advanced MEM gave the best outcome regarding the size and histology of cy reconstituted ovaries at 6 w-ivc (Fig. 5B). Cy reconstituted ovaries reduced their size at the outset of culture, possibly due to ongoing compaction of the aggregates, but they gradually regained their size after 1 w-ive (Fig. 4C). The size of cy reconstituted ovaries cultured in Advanced MEM+10% FBS was consistently larger than that in αMEM containing 10% FBS during the 15-week culture period (Fig. 4C). Cy fetal oocyte development in cy reconstituted ovaries cultured in Advanced MEM+10% FBS under the floating condition was therefore analyzed at 3-week intervals (Fig. 4D). At 3 w-ivc, most germ cells (about 50%) were oogonia expressing NANOG, POU5F1, TFAP2C, and PDPN, while about 30% and about 10% progressed to the RA-responsive (ZGLP1⁺/STRA8⁺) and meiotic (DMC1⁺/γH2AX⁺/SYCP1⁺) stages, respectively (Figs. 4E and F, Fig. 5C). Thereafter, oogonia decreased in number, while meiotic cells increased, albeit with slower kinetics compared to that *in vivo* and in xenotransplantation; nonetheless, oocytes expressing oogenic markers (ZP3, TP63, FIGLA, NOBOX, NLRP5) in primordial follicle-like structures differentiated from 12 w-ivc onward (Figs. 4E and F, Fig. 5C). The morphology of oogonia, and of RA-responsive, meiotic, oogenic, and granulosa cells and the expression/subcellular localization of key markers in these cells in cy reconstituted ovaries *in vitro* were similar to those of the corresponding cell types *in vivo* (Fig. 4E, Figs. 5C and D). The progression of meiotic prophase was examined using the criteria defined in Fig. 1F, which revealed slow but steady progression of the meiotic prophase in cy reconstituted ovaries *in vitro* (Fig. 4G). It was noted that a relatively large fraction of germ cells remained in the pre-leptotene stage from 6 w-ivc to 15 w-ive (Fig. 4G), suggesting that under the current condition, the pre-leptotene to leptotene transition may be a rate-limiting step. These findings demonstrate that cy fetal oocyte development proceeds *in vitro,* with mitotic oogonia differentiating into oocytes that complete the first meiotic prophase in the primordial follicle-like structures over a period of 12 weeks.

### Example 4: Transcriptome dynamics for cy fetal ovary development in vivo, in xenotransplantation, and in vitro

To define cy fetal ovary development *in vivo* as well as in xenotransplantation and under *in vitro* culture in a comprehensive manner, single-cell RNA-sequence (scRNA-seq) analysis of cy fetal ovaries and cy reconstituted ovaries was performed (Table 2). The information on cy/human SC3-seq data (sample information, sequence summary, and data summary) is omitted.

**[Table 2]**

| Information for 10X scRNA-seq datasets. | | | | |
|---|---|---|---|---|
| Sample information, sequence summary, quality control filters, and data summary. | | | | |
| ***in vivo*** | | | | |
| **Reference** | **GEO accession ID** | **Species** | **10X version** | **Sequencer** |
| This study | GSE194266 | *Homo sapiens* | 3.1 | NovaSeq 6000 |
| This study | GSE194266 | *Macaca fascicularis* | 3, 3.1 | NovaSeq 6000 |
| Chitiashvili et al., 2020 | GSE143380 | *Homo sapiens* | 2 | NovaSeq 6000 |
| Niu et al., 2020 | GSE136441 | *Mus musculus* | 2 | NextSeq 500 |
| Zhao et al., 2020 | GSE130212 | *Mus musculus* | 2 | NovaSeq 6000 |
| Ge et al., 2021 | GSE128553 | *Mus musculus* | 2 | HiSeq 2000 |

| | **Low quality cells** | | | **Putative doublets / multiplets** |
|---|---|---|---|---|
| **Reference** | **No. of detected genes (nGene)** | **No. of detected UMIs (nUMI)** | **Mitochondrial gene ratio (%Mt)** | **No. of detected UMIs (nUMI)** |
| This study | **< 2,000** | < 5,000 | > 20 | > 150,000 |
| This study | < 1,000 | < 2,000 | > 20 | > 125,000 |
| Chitiashvili et al., 2020 | < 1,000 | < 2,000 | > 20 | > 125,000 |
| Niu et al., 2020 | < 1,000 | < 2,000 | > 20 | > 75,000 |
| Zhao et al., 2020 | < 1,000 | < 2,500 | > 10 | > 60,000 |
| Ge et al., 2021 | < 1,000 | < 2,500 | > 10 | > 60,000 |

| | **Putative stripped neclei** | **Input** | **Analyzed** | |
|---|---|---|---|---|
| **Reference** | **Mitochondrial gene ratio (%Mt)** | | **All cells** | **Germ cells** |
| This study | < 1 | 5,146 | 4,232 | 504 |
| This study | < 1 | 35,141 | 29,127 | 2,283 |
| Chitiashvili et al., 2020 | < 0.8 | 22,522 | 16,140 | 1,214 |
| Niu et al., 2020 | < 1.2 | 50,794 | 40,703 | 4,028 |
| Zhao et al., 2020 | < 1 | 22,191 | 19,374 | 19,374 |
| Ge et al., 2021 | < 1.2 | 30,272 | 25,504 | 3,954 |

| ***in vitro* culture** | | | | |
|---|---|---|---|---|
| **Reference** | **GEO accession ID** | **Species** | **10X version** | **Sequencer** |
| This study | GSE194266 | *Macaca fascicularis* | 3.1 | NovaSeq 6000 |

| | **Low quality cells** | | | **Putative doublets / multiplets** |
|---|---|---|---|---|
| **Reference** | **No. of detected genes (nGene)** | **No. of detected UMIs (nUMI)** | **Mitochondrial gene ratio (%Mt)** | **No. of detected UMIs (nUMI)** |
| This study | < 1,000 | < 2,000 | > 20 | > 125,000 |

| | **Putative stripped neclei** | **Input** | **Analyzed** | |
|---|---|---|---|---|
| **Reference** | **Mitochondrial gene ratio (%Mt)** | | **All cells** | **Germ cells** |
| This study | < 1 | 1,726 | 937 | 78 |

First, a total of 35,141 single cels isolated from cy fetal ovaries at 8 wpf, 10 wpf, 12 wpf, 16 wpf, and 18 wpf were processed for single-cell cDNA preparation using the 10X Chromium platform. With sequencing, 29,127 cells passed key quality filters [number of genes detected (nGene), total unique molecular identifier (UMI) count, % mitochondrial gene count] and were processed with Scrublet [doublets/multiplets removal algorithm (Wolock SL, et al., Cell Syst, 8: 281-291 e9 (2019))] for subsequent analysis (Table 2). Batch-effect removal from the principal component analysis (PCA) subspace by the fast mutual nearest neighbor (fastMNN) algorithm followed by graph-based clustering using the Louvain algorithm classified these cells into five cell types: DDX4⁺ germ cells (2,283 cells), WT1⁺ pre-granulosa cells (11,986 cells), TCF21⁺ stromal cells (14,028 cells), PECAM1⁺ endothelial cells (673 cells), and TYROBP⁺ blood cells (157 cells) (Figs. 6A-C). In all stages, larger numbers of genes were detected in germ cells than in somatic cells (Fig. 7A). Germ cells were isolated and re-analyzed with PCA, fastMNN, and Louvain clustering, which classified them into 11 clusters after manual removal of putative doublets/multiplets. Based on the expression of key markers, they were annotated as mitotic 1/2/3, pre-leptotene 1/2/3, leptotene, zygotene, pachytene 1/2, and unclassified (most likely apoptotic cells), and some of the pachytene 2 cells were manually classified as diplotene (Fig. 6D, Fig. 7B). The distributions of the cells annotated by scRNA-seq across developmental stages were essentially consistent with those annotated by the IF analysis, except that there were only a few diplotene cells (Fig. 6D, Fig. 7B), which would at least in part be due to an incompatibility of diplotene cells with the 10X platform because of their large size. The numbers of genes detected and total UMI counts were relatively constant from mitotic to zygotene cells (on average, about 7,000 and 50,000, respectively), but were somewhat decreased and more variable in pachytene cells (Fig. 7C).

Mitotic 1/2/3 cells expressed key oogonia markers, including POU5F1, TFAP2C, PDPN, and PRDM1, and consisted predominantly of cells at 8 wpf, but included cells from all developmental stages (Figs. 6D, E and F, Figs. 7B and D). Mitotic 1/2/3 cells were considered to represent cells in the G1, S, G2/M phases of the cell cycle, respectively (Fig. 7E). Pre-leptotene 1 cells weakly expressed POU5F1 and ZGLP1, but did not show typical meiotic markers such as DMC1, PRDM9, and SYCP1, and consisted of cells from 8 wpf, 10 wpf, 12 wpf, and 16 wpf. They were in the G1 phase of the cell cycle (Figs. 6D and F, Figs. 7D and E). Pre-leptotene 2/3 cells expressed ZGLP1, REC8, and SYCP3, but were negative/very weak for DMC1, PRDM9, and SYCP1, consisted of cells from all developmental stages, and were in the S and G2 phases of the cell cycle, respectively (Figs. 6D and F, Figs. 7D and E). Thus, pre-leptotene 2 cells are those undergoing meiotic DNA replication. Leptotene and zygotene cells consisted of cells mainly after 12 wpf, and expressed REC8, SYCP2, and SYCP3 at a higher level than pre-leptotene cells, with zygotene cells up-regulating DMC1, PRDM9, and SYCP1 (Figs. 6D and F, Fig. 7D). Pachytene 1/2 cells also consisted of cells mainly after 12 wpf, repressed ZGLP1 and REC8, and expressed SYCP1/2/3, DMC1, and PRDM9, with pachytene 2 cells initiating the down-regulation of these genes and up-regulation of FIGLA (Figs. 6D and F, Fig. 7D). Diplotene cells (ZP3⁺/GDF9⁺/NOBOX⁺ cells) consisted of cells at 16 wpf and 18 wpf, down-regulated meiotic markers, and expressed key genes for oocyte development, such as FIGLA and NLRP5 (Figs. 6D and F, Fig. 7D). A pseudotime analysis for developmental trajectory by Monocle 3 (Cao J, et al., Nature, 566: 496-502 (2019)) gave an outcome consistent with the above results (Fig. 6G).

Highly variable genes (HVGs) were identified among the 11 clusters [top 2,000 HVGs minus genes with low-expression levels (cluster average expression log (ssUMI+1) ≤ 0.5): 1,481 genes]. The lists of the 1,481 identified genes, their normalized expression values, and GO analysis of HVG are omitted.

Unsupervised hierarchical clustering (UHC) classified such genes into 8 clusters (Fig. 6H): the cluster 1 genes were expressed highly in mitotic 1/2/3 cells and down-regulated acutely after the pre-leptotene 1 stage, and were enriched in genes with gene ontology (GO) functional terms for "somatic stem cell population maintenance" (LIN28A, DPPA4, NANOG, KLF4, etc.) and "developmental process involved in reproduction" (PRDM1, UTF1, TFAP2C, NANOS3, SOX15, etc.). The cluster 2 genes showed an expression profile associated with cell-cycle progression (expressed in mitotic 2/3, pre-leptotene 2/3) and were indeed enriched for "mitotic cell cycle" and "meiotic cell cycle process" (TOP2A, MCM3/10, MKI67, CCNA2/B1/B2, PCNA, etc.) (Fig. 6H, Fig. 7E). The cluster 3 genes were expressed from pre-leptotene 1 to zygotene, and were enriched for "regulation of transcription, DNA-templated" (STRA8, ZGLP1, ANHX, KDM6B, etc.), and interestingly, "pattern specification process" (many HOX genes, SIX1, TBX3, GLI3, PBX1, etc.). The cluster 4 genes were gradually up-regulated from pre-leptotene 1 onwards and were enriched for "meiotic cell cycle" (TDRD1/12, RAD50, EHMT2, MSX1, PLD6, etc.) and "reproductive system development" (HOXA9/A10, SERPINE2/F1, CHD7, etc.). The cluster 3 and 4 genes, particularly those expressed in pre-leptotene 1/2, would include the oogenic fate determinant. The cluster 5 and 6 genes were expressed from leptotene to pachytene 2 (cluster 5) or diplotene (cluster 6), and included key genes for meiotic recombination ["synapsis/double-strand break repair/reciprocal DNA recombination/meiotic cell cycle" (cluster 5: SYCP1, SYCE1/2/3, MSH4/5, MEIOB/C, TEX11/15, PRDM9, SPO11, MEIOSIN, etc.; cluster 6: SYCP2, ASZ1, MAEL, TEX12, MEIKIN, etc.)]. The cluster 7 and 8 genes were strongly up-regulated in diplotene and were enriched for "reproductive process" and "oogenesis" (NPM2, BMP5, GDF9, ZP2/3, SOHLH1, FIGLA, NOBOX, etc.). The correlation analysis using the same genes revealed that the 11 clusters can be classified into 4 groups with higher correlations [mitotic 1-to-pre-leptotene 1, pre-leptotene 1-to zygotene (corresponding to RA-responsive), zygotene-to-pachytene 2 (corresponding to meiotic), pachytene 2-to-diplotene (corresponding to oogenic)], with 3 clusters (pre-leptotene 1, zygotene, pachytene 2) playing a transitional role between the groups (Fig. 6I), providing high-resolution transcriptome categorization for primate fetal oocyte development.

A total of 937 quality-filtered single-cell transcriptomes were generated from cy reconstituted ovaries at 12 and 15 w-ive with 10X Chromium (73 germ cells, 702 (pre-)granulosa cells, and 118 stromal cells) and were compared with those of cy fetal ovarian cells *in vivo.* PCA and fastMNN followed by Louvain clustering revealed that cy germ cells in cy reconstituted ovaries *in vitro* exhibited transcriptomes concordant with those of cy oocytes ranging from the pre-leptotene to pachytene stage (Fig. 6J). To gain further insights into the transcriptome dynamics associated with cy fetal oocyte development in cy reconstituted ovaries, single cells with germ-cell appearance were manually picked up from cy reconstituted ovaries at 3/6/9/12/15 w-ivc and w-ptp, as well as from oocytes at 12/16/18 wpf and ovaries in adult individuals, and scRNA-seq analysis was performed with single-cell mRNA 3-prime end sequencing (SC3-seq) (Nakamura T, et al., Nucleic Acids Res, 43: e60 (2015)) (Figs. 6K and L). UHC and PCA classified 172 quality-filtered DDX4⁺ single cells (including 19 germ cells at 6/7 wpf in (Okamoto I, et al., Science, 374: eabd8887 (2021)) into four major groups corresponding to mitotic, RA-responsive, meiotic, and oogenic cells, and *in vivo,* xeno-transplanted, and *in vitro* cells were included in all groups, with intermingling transcriptome profiles within the groups (Figs. 6K and L). Collectively, these results led us to conclude that cy fetal germ cells undergo appropriate transcriptome maturation in cy reconstituted ovaries *in vitro.*

The development of WT1⁺ cy pre-granulosa cells was also examined *in vivo* and *in vitro.* Using the same analysis as for germ cells, cy (pre-)granulosa cells *in vivo* were classified into 12 clusters after manual removal of putative doublets/multiplets (Fig. 8A). Analysis of the cluster distributions across developmental stages showed that most clusters (clusters 1, 2, 3, 4, 5, 6, 7, 9, 11, and 12) were present as early as 8 wpf, while clusters 8 and 10 emerged from 10 and 16 wpf onwards, respectively (Figs. 8A-C). Genes with highly variable expression were identified among the 12 clusters [top 1,500 HVGs minus genes with low-expression levels (cluster average expression log (ssUMI+1) ≤ 0.5): 788 genes]. The lists of the 712 identified genes, their normalized expression values, and GO analysis of HVG are omitted.

UHC using these genes revealed that cells in clusters 2 and 3 were pre-granulosa cells in the S phase of the cell cycle, while those in cluster 1 were in the G2/M phase; moreover, the cells in cluster 1 were abundant in the early stages (8/10/12 wpf) and, like the cells in cluster 4, were enriched with genes for "cell migration" and "cytoskeleton organization" (Figs. 8A-D). Cells in clusters 11 and 12 were also abundant in the early stages (8/10/12 wpf) and expressed genes for "steroid biosynthetic process" and "reproductive system development," and thereby represented a unique pre-granulosa cell subtype (Figs. 8A-D). The RNA velocity calculation (Bergen V, et al., Nat Biotechnol, 38: 1408-1414 (2020)) and partition-based graph abstraction (PAGA) (Wolf FA, et al., Genome Biol, 20: 59 (2019)) suggested that pre-granulosa cell differentiation proceeded from cluster 4 to 5 to 7 to 9 or from 4 to 5 to 6 to 8 to 10 (Figs. 8A and B), and cells in cluster 10, which emerged from 16 wpf onwards, were enriched with genes for "extracellular matrix organization" (COL1A2/3A1/4A3/6A1/6A2/14A1, LAMAA4/B1, ITGA1/A2/A6, TIMP2, SULF1, etc.) and "Notch signaling pathway" (NOTCH3, JAG1, HEY2, NR1H4, HES1), and would therefore represent granulosa cells composing primordial follicles (Figs. 8D and E) (Li L, et al., Cell Stem Cell, 20: 858-873 e4 (2017)). Combined analysis of the transcriptomes of WT1⁺ pre-granulosa cells in cy reconstituted ovaries *in vitro* revealed that most WT1⁺ cells at 12 and 15 w-ive formed a cluster (cluster 12) close to/merged with a cluster of granulosa cells in the primordial follicles at 16 and 18 wpf (cluster 11), indicating that pre-granulosa cells at 8 wpf underwent appropriate differentiation into follicular granulosa cells (Figs. 8F and G). It was noted, however, that follicular granulosa cells *in vitro* showed a signature for activated granulosa cells, including ID2/3/4 up-regulation (i.e., activation of BMP signaling) and HEY2 up-regulation and HEY1/HEYL down- regulation (i.e., regulation of JAG1-NOTCH2/3 signaling) (Fig. 8G) (Vanorny DA, and Mayo KE, Reproduction, 153: R187-R204 (2017), Zhang Y, et al., Mol Cell, 72: 1021-1034 e4 (2018)), suggesting a possibility that granulosa-cell development in culture proceeds to an activated primary follicle stage.

### Example 5: Reconstitution of human fetal oocyte development in vitro

Having established a condition for cy fetal oocyte development *in vitro,* it was explored whether human fetal oocyte development can also be reconstituted *in vitro.* Two aborted human female fetuses at 11 weeks of gestation (11W) were obtained, and their gonads were isolated and processed for histological analysis, human reconstituted ovary formation, and single-cell transcriptome analysis. Additionally, primordial follicles from a human adult ovary were isolated, and their oocytes were processed for single-cell transcriptome analysis. All human samples were obtained upon appropriate informed consent (see <Materials and Methods>). Consistent with previous reports (Li L, et al., Cell Stem Cell, 20: 858-873 e4 (2017)), human fetal ovaries at 11W were abundant with oogonia bearing a round nucleus with pale hematoxylin staining and a prominent nucleolus, but were also populated with cells with a larger nucleus with a more condensed chromatin appearance (Fig. 9A). Accordingly, about 85% of DDX4⁺ germ cells were POUSF1⁺ and a small fraction of them (about 3%) showed expression of early meiotic markers such as SYCP3, γH2AX, and DMC1, but no cells expressed SYCP1 (Figs. 9B and D), demonstrating that most germ cells in human ovaries at 11W are oogonia.

Under the air-liquid interface condition, cells migrated out of human reconstituted ovaries (about 50,000 ovarian cells) and human reconstituted ovaries became flattened out and collapsed over an extended culture period; this was similar to the findings in cy reconstituted ovaries, but dissimilar to mouse reconstituted ovaries (Fig. 5E). Human reconstituted ovaries were therefore cultured under the floating condition defined for cy reconstituted ovaries and their properties were examined at 7/8 and 14 w-ivc. At 7 w-ivc, human reconstituted ovaries showed an integrated structure, although like cy reconstituted ovaries, the central areas exhibited a necrotic appearance (Fig. 9A). Histological sections revealed that the cortical areas were full of germ cells bearing oogonia-like or early meiotic oocyte- like morphology (Fig. 9A). In good agreement with this result, about 30% of DDX4⁺ germ cells were POU5F1⁺, about 50% were SYCP3⁺, and about 30% were γH2AX⁺/DMC1⁺, while only about 2% were SYCP1⁺ (Figs. 9C and D). At 14 w-ivc, human reconstituted ovaries remained integrated and their cortexes were abundant with germ cells bearing a large nucleus with condensed, thread-like chromatin and, remarkably, a number of primordial follicle-like structures (Fig. 9A). Consistent with these observations, the ratio of DDX4⁺/POU5F1⁺ cells decreased to about 10%, while about 30% became SYCP1⁺ and about 3% were ZP3⁺/TP63⁺ (Figs. 9C and D). The morphology of meiotic and primordial follicle-like cells and the expression/subcellular localization of key markers in these cells were similar to those of corresponding *in vivo* cell types in cynomolgus monkeys and humans (Figs. 1E and 9C) (Baker TG, Proc R Soc Lond B Biol Sci, 158: 417-33 (1963)).

Single-cell transcriptome analysis by SC3-seq of 120 quality-filtered single cells (human fetal ovaries at 11W: 33 cells; human adult ovaries: 4 cells; human reconstituted ovaries at 8 w-ivc: 39 cells; human reconstituted ovaries at 14 w-ivc: 44 cells) classified them into four cell types: mitotic, RA-responsive, meiotic, and oogenic cells (Figs. 9E and F). Consistent with the histological and IF analyses, most single cells picked up from dissociated human fetal ovaries at 11W were mitotic oogonia expressing NANOG, POU5F1, TFAP2C, and PRDM1 (19/33) or RA-responsive cells expressing ANHX, REC8, and ASB9 (14/33), whereas those from human reconstituted ovaries at 8 w-ivc contained all four cell types (mitotic: 16/39; RA-responsive: 13/39; meiotic: 7/39; oogenic: 3/39) and those at 14 w-ivc were predominantly meiotic cells expressing DMC1, RAD51AP2, and SYCP1 or oogenic cells with ZP3, NLRP5, TP63, and NOBOX (mitotic: 0/44; RA-responsive: 3/44; meiotic: 21/44; oogenic: 20/44) (Figs. 9E and F). Notably, the oogenic cells isolated from human reconstituted ovaries were clustered closely with adult oocytes in the primordial follicles (Figs. 9E and F), indicating a proper progression of human oocyte development in human reconstituted ovaries *in vitro.* To substantiate this point further and complement the lack of human fetal ovary samples at later stages, SC3-seq data of human and cy germ cells were merged by canonical correlation analysis (CCA) (Stuart T, et al., Cell, 177: 1888-1902 e21 (2019)), and PCA was performed, which revealed a highly parallel transcriptomic progression of human oocytes in human reconstituted ovaries *in vitro,* and in cy oocytes *in vivo* and cy reconstituted ovaries *in vitro* (Fig. 9G). Taken together, these results indicated that human oocyte development can also be reconstituted *in vitro,* at least up to the oocytes that complete the meiotic prophase I in the primordial follicles.

### Example 6: Primate-specific program for fetal oocyte development

In mammals, germ-cell development proceeds with conserved cellular events, which include PGC specification from pluripotent embryonic precursors, epigenetic reprogramming, germ-cell sex determination, and epigenetic programming coupled with (in females) or followed by (in males) meiotic divisions to generate sexually dimorphic haploid gametes. On the other hand, the precise cellular dynamics and molecular mechanisms underlying such conserved events appear to vary widely among species (Saitou M, and Hayashi K, Science, 374: eaaz6830 (2021)). To gain insight into the primate-specific program associated with fetal oocyte development, relevant transcriptome datasets among mice, monkeys, and humans were compared. Cynomolgus monkey and human datasets created in this Example were merged with four public datasets [one for humans (Chitiashvili T, et al., Nat Cell Biol, 22: 1436-1446 (2020)) and three for mice (Ge W, et al., Cell Mol Life Sci, 78: 695-713 (2021), Niu W, and Spradling AC, Proc Natl Acad Sci USA, 117: 20015-20026 (2020), Zhao ZH, et al., FASEB J, 34: 12634-12645 (2020))] using CCA, Louvain clustering was performed, and the fetal oocyte developmental stage of the 15 clusters across the three species was defined based on the expression of the relevant genes (mitotic 1-4, pre-leptotene 1-3, leptotene 1-3, zygotene 1/2, pachytene 1/2, diplotene) (Fig. 10A, Table 2). The distributions of the cells with defined developmental stages in each developmental time point were in accord with previous observations (Figs. 10B and C, Fig. 11A) (Chitiashvili T, et al., Nat Cell Biol, 22: 1436-1446 (2020) ) (Ge W, et al., Cell Mol Life Sci, 78: 695-713 (2021), Niu W, and Spradling AC, Proc Natl Acad Sci USA, 117: 20015-20026 (2020)).

Pseudotime analysis by Monocle 3 revealed that in humans and monkeys, the mitotic to pre-leptotene transition, i.e., the entry into meiosis, produced relatively large transcriptomic alterations, whereas in mice, such transition involved minor change, but the subsequent transitions gave rise to major alterations (Fig. 10D). HVGs during fetal oocyte development in each species were identified, among which 237 genes were orthologous and common to all three species. The correlation analysis of their expression in each developmental stage revealed a major distinction between the mitotic and pre-leptotene cells in humans and monkeys, but not in mice (Fig. 10E). Furthermore, the expression properties of each cluster were relatively well conserved between humans and monkeys but were divergent in mice (Fig. 10E). A previous study has shown that during the meiotic prophase for spermatogenesis in humans, monkeys, and mice, as meiosis progresses, the contribution to the overall expression of one-to-one-to-one (1-1-1) orthologous genes declines, whereas that of species-specific genes increases in humans and mice (no clear conclusion was reached for monkeys due to the low level of gene annotation), arguing for species-specific specialization for male meiotic prophase I (Lau X, et al., Dev Cell, 54: 548-566 e7 (2020)). The same analysis was performed for female meiotic prophase I. In clear contrast to the findings in males, as meiosis progressed in females, the contribution to the overall expression of 1-1-1 orthologues gradually increased, whereas that of the species-specific genes was consistently low (Fig. 11C). Thus, during oogenesis, in humans and monkeys, the mitotic-to-meiotic transition involves more drastic transcriptomic changes than in mice, and overall transcriptomic divergence among the three species derives from the distinct usage of orthologous genes.

Furthermore, primate-specific genes that exhibit dynamic changes during fetal oocyte development were identified. Based on the SC3-seq analysis in Fig. 9G, differentially expressed 1-1 orthologues between the two successive cell types [log2 (normalized read counts + 1) > 3, fold-change > 2] were identified, common genes in humans and monkeys (397 genes) were extracted, and their expression changes in the 10X datasets in fetal oocyte developmental stages as defined in Fig. 10A were examined. The list of 397 genes and their normalized expression values in human/monkey/mouse 10X scRNA-seq data is omitted.

The expression profiles of 130 of the 397 genes were well conserved among the three species, and a notable trend was observed: genes expressed in mitotic cells continued to be expressed in pre-leptotene/leptotene cells, and those expressed in meiotic cells showed up-regulation in relatively late stages in mice (Fig. 12A). These genes included well-known genes involved in fetal oocyte development, including PRDM1, TFAP2C, NANOS3, TCL1A, SOX15, DPPA3 (mitotic), HORMAD1, REC8, STAG3, SMC1B (leptotene onwards), SYCP1, SYCP2, SYCE3, HORMAD2, MEIOB, MEIOC, TEX12, SPO11 (zygotene onwards), NPM2, ZP2, NOBOX, FIGLA, GDF9, NLRP5, and TP63 (pachytene 3/diplotene) (Fig. 12A). On the other hand, 83 genes showed specific expression changes in humans and monkeys but not in mice, and these included, among other genes, PIM2, THY1, MMP2, SOX17, TFCP2L1 (mitotic), PBX1, SKAP2, KDM1B, HOXA3/B3, PAX6, MORC4 (pre-leptotene/leptotene), DMRTB1, CLGN, GSAP, SPAG6, UPB1, CABYR (leptotene onwards), SLCO4A1, CSF1R, TMEM163, KPNA7, ARHGEF33/GAP18, SERPINF1, and LMOD3 (pachytene 2/diplotene) (Fig. 10F, Fig. 12B). Among these, the expression of PAX6, a homeobox TF involved in key cell-fate specification processes including eye development (Jordan T, et al., Nat Genet, 1: 328-32 (1992), Walther C, and Gruss P, Development 113: 1435-49 (1991)), CLGN, a chaperone that is specifically expressed in the endoplasmic reticulum (ER) of spermatogenic cells and plays a key role in sperm binding to ZP (Ikawa M, et al., Nature, 387: 607-11 (1997)), and LMOD3, an actin filament-nucleating protein expressed in skeletal and cardiac muscle (Yuen M, et al., J Clin Invest, 124: 4693-708 (2014)), was verified. IF analysis revealed that PAX6 showed specific and strong expression in TFAP2C⁻ germ cells at an early stage of the meiotic prophase in cynomolgus monkeys and human reconstituted ovaries (Figs. 10G and H); CLGN was expressed and localized in the cytoplasm, most likely in the ER, in TFAP2C⁻ germ cells in cynomolgus monkeys and human reconstituted ovaries, and in human juvenile primordial follicles (Figs. 10G-I); and LMOD3 exhibited specific expression in primordial follicles in both cynomolgus monkeys and humans, with meshwork-like localization in the cytoplasm (Fig. 12C). Interestingly, LMOD3 showed higher enrichment in the cytoplasm facing toward the ovarian medulla in primordial follicles in human juveniles (Fig. 12C).

### Example 7: Conserved activities of X chromosomes

In mammals, female cells bear two X chromosomes (Xs), while male cells have one X and one Y chromosome. In females, one X is inactivated (Xi) in a random manner in early post- implantation embryos (X-chromosome inactivation: XCI) to compensate for the X-linked gene dosage with males (Lyon MF, Nature, 190: 372-3 (1961), Zylicz JJ, and Heard E, Annu Rev Biochem, 89: 255-282 (2020)). In somatic cells, one active X chromosome (Xa) is hyperactivated to compensate for the X chromosome: autosome (X:A) gene dosage (X-chromosome up-regulation: XCU) in both females and males (Deng X, et al., Nat Rev Genet, 15: 367-78 (2014), Ohno S, Springer-Verlag, Berlin (1967)). On the other hand, in female germ cells, Xi is reactivated (X-chromosome reactivation: XCR) (Chitiashvili T, et al., Nat Cell Biol, 22: 1436-1446 (2020), Guo F, et al., Cell, 161: 1437-52 (2015), Monk M, et al., J Embryol Exp Morphol, 63: 75-84 (1981), Okamoto I, et al., Science, 374: eabd8887 (2021), Sugimoto M, and Abe K, PLoS Genet, 3: e116 (2007), Tang WW, et al., Cell, 161: 1453-67 (2015), Vertesy A, et al., Nat Commun, 9: 1873 (2018)), and it has been shown that germ cells exhibit a non-canonical X-chromosome dosage compensation in mice and humans (Sangrithi MN, et al., Dev Cell, 40: 289-301 e3 (2017)). However, another study reached a different conclusion (Chitiashvili T, et al., Nat Cell Biol, 22: 1436-1446 (2020)).

The X chromosome activities during fetal oocyte development in the three species were therefore sought to be compared. The X:A ratio of gene-expression levels during mouse germ-cell development was first examined based on bulk RNA-seq data, which ensures high quantitative performance. Mouse bulk RNA-seq data information (sample information, sequence summary, and data summary) is omitted. In gonadal somatic cells, the X:A ratio was slightly below 1 (about 0.9), indicating that XCU occurs, if not fully, on a single Xa (Fig. 13A). Information on X:A ratio analysis using the 10X/SC3-seq/bulk RNA-seq dataset is omitted.

In female germ cells, the X:A ratio was below 1 (about 0.9) until E12.5 during their mitotic expansion, exceeded 1 (about 1.1) from E13.5 to E18.5 during the progression of meiotic prophase I, and was down-regulated to about 1 at postnatal day (P) 0 and 1 in the primordial follicles (Fig. 13A). Given that both X chromosomes are progressively activated in germ cells by E14.5 (Sugimoto M, and Abe K, PLoS Genet, 3: e116 (2007)), this indicates a mild XCU during the meiotic prophase and its erasure in the primordial follicles. In male germ cells, the X:A ratio was about 0.83 at E11.5, and it decreased to about 0.65 at E12.5 and remained at about 0.7 in the mitotic arrest phase (Fig. 13A), indicating that XCU erasure also occurs in males. Interestingly, a mild up-regulation of Xist (X inactive specific transcript), the long non-coding RNA inducing a cascade of repressive events for XCI in mice (Brockdorff, Ashworth et al., 1992, Marahrens, Panning et al., 1997, Penny, Kay et al., 1996), was detected in the primordial follicles, while it was essentially repressed in male germ cells (Fig. 13A). The timing of the onset and erasure of a mild XCU in females was different from, but that of the XCU erasure in males was consistent with, a previous study (Sangrithi MN, et al., Dev Cell, 40: 289-301 e3 (2017)). The X:A ratio during cy and human fetal oocyte development *in vivo,* in xenotransplantation, and *in vitro* was next examined based on the SC3-seq datasets. During female cy embryonic development, XCI occurs coincidentally with XCU in somatic cells, and cy germ cells initiate XCR upon their specification and complete it by about E50 (Okamoto I, et al., Science, 374: eabd8887 (2021)). It was found that *in vivo,* despite XCR, the X:A ratio was below 1 in mitotic and RA-responsive cells (about 0.90 and about 0.68, respectively), increased to about 1 in meiotic cells, and then decreased below 1 (about 0.71) in oogenic cells (Fig. 13B). XIST was expressed at a high level in mitotic germ cells, was repressed in RA-responsive cells, exhibited an up-regulation in meiotic cells, and was repressed again in oogenic cells (Fig. 13B). The dynamics of the X:A ratio and XIST expression were similar in fetal oocyte development in xenotransplantation and *in vitro* as well as during human fetal oocyte development *in vivo* and *in vitro* (Figs. 13B and C). The X:A ratio dynamics in the three species were then analyzed based on the 10X datasets, which revealed that although the dynamic range was narrower, similar X:A ratio dynamics were exhibited in the three species: the ratio was below 1 in mitotic and early meiotic (pre-leptotene) oocytes, increased to about 1 in late meiotic (zygotene/pachytene) oocytes, and decreased below 1 in oocytes in the primordial follicles (diplotene oocytes) (Fig. 13D, Fig. 12D). XIST showed a similar expression profile in humans and monkeys: it was expressed at a significant level in mitotic cells (similar to that in gonadal somatic cells) and then downregulated thereafter, whereas in mice, it was essentially repressed in mitotic and meiotic cells and showed a mild up-regulation in diplotene oocytes (Fig. 13D, Fig. 12D). These findings are in agreement with the findings by Chitiashvili et al., who reported the X:A dynamics during human fetal oocyte development (Chitiashvili T, et al., Nat Cell Biol, 22: 1436-1446 (2020)), but are distinct from those of Sangrithi et al., who reported a mild XCU in human mitotic oogonia (Sangrithi MN, et al., Dev Cell, 40: 289-301 e3 (2017)). Based on the collective data obtained in this Example, it is concluded that although some levels of quantitative variability exist depending on the measurement methodology, on the whole, the X-linked gene dosage is regulated in a similar manner in humans, monkeys, and mice, and fetal oocyte development proceeds with two Xa and with little, if any, XCU.

### Example 8: Evaluation of effects of addition of steroid hormones to culture medium

An experiment in which steroid hormones were added to the above-described culture medium was carried out. The "steroid hormones" here refer to progesterone, cortisol, dehydroepiandrosterone, and dehydroepiandrosterone sulfate, which is a sulfate conjugate thereof, whose concentration in fetal cynomolgus monkey blood can be measured. In the experiment, the following substances were all added at concentrations equivalent to physiological concentrations in humans and cynomolgus monkeys: progesterone, 200 ng/mL; cortisol, 100 ng/mL; dehydroepiandrosterone, 50 nM; and dehydroepiandrosterone sulfate, 10 µM. On the other hand, none of these hormones were added to steroid hormone-free medium. The same applies to the following examples.

In the case of culture in the steroid hormone-free medium, it took 12 weeks for primordial follicle formation to occur, and no primordial follicle formation was observed in the reconstituted ovary at 9 weeks of culture. On the other hand, in the steroid hormone addition group, the formation of primordial follicles was observed at 9 weeks of culture. Therefore, it is conceivable that the addition of steroid hormones facilitates the differentiation and maturation of oocytes, producing primordial follicles in a short period of time (Figs. 14 and 15).

### Example 9: Evaluation of effects of addition of retinoic acid (RA) to culture medium

The retinoic acid (RA)-free group and the retinoic acid addition group were compared under FBS-free conditions. The addition group was examined at different RA concentrations of 10 nM/100 nM/500 nM/1 µM. In the RA addition groups, meiotic markers became positive and oogonia markers became negative earlier than in the free groups, regardless of the concentration (Figs. 16 and 17). That is, it is conceivable that the addition of RA to this culture system will have the effect of facilitating differentiation of oogonia into oocytes, and the effect of facilitating the initiation and progression of meiosis. These effects were similar at all concentrations, but as the concentration increased, the increase in the size of the reconstituted ovary was further inhibited, and thus it is conceivable that 10 nM, at which a sufficient effect can be obtained, is an appropriate concentration when RA is added.

### Example 10: Evaluation of effects of simultaneous addition of steroid hormones and retinoic acid (RA) to culture medium

In this example, it was also confirmed that primordial follicles were more efficiently produced in the steroid hormone addition group and the retinoic acid (RA)-free group (10 nM/FBS(-)) than in the steroid hormones and retinoic acid-free group (Fig. 18). Under the condition that steroid hormones and RA (10 nM/FBS(-)) were added simultaneously, a greater number of primordial follicles were produced (Fig. 18). Therefore, it was suggested that steroid hormones and RA synergistically increase the efficiency of primordial follicle production.

### <Materials and Methods>

### (Animals)

All animal experiments were performed with the ethical approvals of Kyoto University and Shiga University of Medical Science. ICR and immunodeficient KSN/Slc mice were purchased from SLC (Shizuoka, Japan). The C57BL/6N;mvh-RFP^{Tg/Tg} (VR) and Dppa3(Stella)-EGFP^{Tg/Tg} transgenic mice were established as described previously (Imamura M, et al., Mol Reprod Dev, 77: 802-11 (2010), Miyauchi H, et al., EMBO J, 36: 3100-3119 (2017), Payer B, et al., Genesis, 44: 75-83 (2006), Seki Y, et al., Development, 134: 2627-38 (2007)). Mice were housed in a specific pathogen-free animal facility under a 14-h light/10-h dark cycle. For the animal experiments using cynomolgus monkeys (cy), the procedures for oocyte collection, intra-cytoplasmic sperm injection, pre-implantation embryo culture, and embryo transfer into foster mothers were as described previously (Yamasaki J, et al., Theriogenology, 76: 33-8 (2011)). Pregnancy was checked by transabdominal fetal echography at 4 wpf, and the blood plasma of the pregnant monkey was collected followed by the extraction of fetal cell-free DNA. To minimize the number of cy fetuses used, cell-free DNA in maternal plasma was purified by a QIAamp DNA Mini Kit (QIAGEN, 51304), and the sex of fetuses was determined by real-time PCR targeted for SRY and DYS14 genes (Yasmin L, et al., Comp Med, 65: 70-6 (2015)). For collecting the cy fetuses, cesarean sections were performed under isoflurane and ketamine-based general anesthesia. The sex of the fetus was further confirmed by checking the gross appearance of its gonads or by PCR for UTX/UTY genes (Villesen P, and Fredsted T, BMC Ecol, 6: 8 (2006)).

### (Collection of human embryo and ovary samples)

Human fetal or adult ovaries were collected after informed consent from participants undergoing elective abortion or gynecological procedures. All experimental procedures were approved by the Institutional Review Board at Kyoto University (approval nos. G1047 and G1192). The sex of the fetus was determined by sex-specific PCR for the ZFX/ZFY and SRY loci (Josef B, and Adam K, Folia Zoologica, 52: 269-274 (2003)).

### (Histological analyses)

For immunohistochemical analyses, fetal ovaries or reconstituted ovaries were fixed in 4% paraformaldehyde/PBS (Sigma-Aldrich, 158127) at 4°C for 30 min or overnight, embedded in paraffin wax (Leica, 39601006) or OCT compound (Sakura Finetek, 4583), and sectioned at a thickness of 5 or 8 µm. After deparaffinization, the sections were pre- treated with an antigen retrieval reagent, HistVT One (Nacalai Tesque, 06380-76), at 90°C for 40 min. Sections were treated with blocking solution (1% bovine serum albumin (Sigma-Aldrich, A7030), 5% normal donkey serum (JIR, 017-000-121), 0.1% Tween20, and 1X phosphate buffered saline) for 30 min at room temperature, and incubated with primary antibodies in blocking solution at 4°C overnight. The slides were further incubated with Alexa Fluor-conjugated secondary antibodies. Fluorescence images were captured using a laser-scanning confocal microscope (OLYMPUS, FV1000; ZEISS, LSM780). For the quantitative analysis of each marker, germ cells marked with DDX4 expression were analyzed. To define the "inner" and "outer" cortex for fetal ovaries at 10-18 wpf, the ovarian cortex was divided into two at the middle of the cortex. Hematoxylin and eosin staining were performed using the standard procedure.

### (Staging of meiosis in ovarian sections)

Germ cells expressing DDX4 in fetal ovarian sections were classified into 7 stages by the pattern of nuclear staining of DAPI, SYCP3, and DMC1. The stages were defined as follows. Mitotic: cells without any SYCP3 and DMC1 signal. Pre-leptotene: numerous short nuclear threads with low SYCP3 signal. Leptotene: numerous short nuclear threads with low SYCP3 and DMC1 signals. Zygotene: numerous long thin threads of SYCP3 staining with many DMC1 dots. Early pachytene: thick threads of SYCP3 staining with many DMC1 foci. Late pachytene: thick threads of SYCP staining with less than one DMC1 dot on each chromosome. Diplotene: numerous short nuclear threads with faint or no SYCP3 signal. Diplotene cells were distinguished from pre-leptotene ones by their size and histological morphology.

### (Dissociation of cy/human fetal ovarian tissues and cy/human reconstituted ovaries)

Fetal ovaries collected from cy fetuses at 8-18 wpf were carefully dissected out in chilled PBS. Fetal ovaries at 8-14 wpf were minced with scissors in 0.05% trypsin/EDTA/PBS solution (GIBCO, 15400-054), then incubated for 15 min at 37°C with gentle pipetting every 5 min. Dissociation medium [DMEM (GIBCO, 10313021) containing 10% FBS, 2 mM L-glutamine (GIBCO, 25030081), 1X penicillin/streptomycin (GIBCO, 15070063), and 100 µg/mL DNaseI (MERCK, DN25)] was added for further dissociation. Dissociated cells were used for single-cell RNA-seq analyses, or resuspended in CELLBANKER 1 plus (ZENOGEN PHARMA, CB021) and stored at -80°C. Human fetal ovaries at 11 weeks of gestation were also dissociated and cryopreserved using the same procedures.

For fetal ovaries at 16 and 18 wpf, minced tissues were incubated in Accumax (Innovative Cell Technologies, AM105) for 40 min at 37°C with gentle pipetting every 5 min. Dissociation medium was added for further dissociation. For SC3-seq analysis, cells were picked up under the inverted microscope (OLYMPUS, IX71) to exclude multiplet cells. For 10X experiments, cell suspensions were further treated with TryPLE Express (1 X) (GIBCO, 12604021) for 5 min at 37°C to prepare the single-cell suspension.

For dissociating cy/human reconstituted ovaries, tissues were incubated with TryPLE Express (1 x) (GIBCO, 12604021) for 5 min. After adding dissociation medium, cells were processed for single-cell analyses.

### (In vitro culture of cy/human reconstituted ovaries under an air-liquid interface culture condition)

The cy fetal ovarian cells (50,000 cells/well) at 8 wpf were plated on wells of Nunclon Sphera-Treated U-Shaped-Bottom 96-well plates (NUNC, 174925) and cultured under a floating condition in αMEM medium containing 10% FBS, 2 mM L-glutamine (GIBCO, 25030081), 1X NEAA (GIBCO, 11140050), 1 mM sodium pyruvate (GIBCO, 11360070), 1X penicillin/streptomycin (GIBCO, 15070063), 100 µM 2-mercaptoethanol (GIBCO, 21985023), and 10 µM Y-27632 dihydrochloride (TOCRIS, 1254). After 2 days, using a glass capillary, the cy reconstituted ovaries were transferred onto a Transwell-COL membrane insert (Corning, 3495) soaked in αMEM (GIBCO, 12571063) containing 10% FBS, 2 mM L- glutamine (GIBCO, 25030081), 150 µM ascorbic acid (Sigma-Aldrich, A4403), 1X penicillin/streptomycin (GIBCO, 15070063), and 55 µM 2-mercaptoethanol (GIBCO, 21985023). Half the medium was changed every two days. Reconstituted ovaries were cultured at 37°C under an atmosphere of 5% CO₂ in air. The human reconstituted ovary was generated from human fetal ovarian cells at 11 weeks of gestation and cultured under the same conditions, except that Advanced MEM was used as the basal medium.

To assess the coating condition, Preset VECELL membranes (VECELL, PSVC12-10) were coated with human collagen type I (Millipore, CC050), type III (Corning, 354244), type IV (Corning, 354245), type V (Corning, 354246), and type VI (Corning, 354261), human fibronectin (Corning, 354008), iMatrix (Nippi, 892014), laminins (111/121/211/221/411/421/332/521) (VERITAS, BLA-LNKT-0201), growth factor-reduced Matrigel (Corning, 356230), and their combinations prior to IVC following the manufacturers' coating protocols.

### (In vitro culture of cy/human reconstituted ovaries under the floating condition)

A cy reconstituted ovary was generated from the 8 wpf fetal ovarian cells (50,000 cells/well) under a floating condition in Advanced MEM (GIBCO, 12492013) containing 10% FBS, 2 mM L-glutamine (GIBCO, 25030081), 1X NEAA (GIBCO, 11140050), 1 mM sodium pyruvate (GIBCO, 11360070), 1X penicillin/streptomycin (GIBCO, 15070063), 100 µM 2-mercaptoethanol (GIBCO, 21985023), and 10 µM Y-27632 dihydrochloride (TOCRIS, 1254). After two days, the cy reconstituted ovary was transferred into another well of a U-bottom culture plate using a glass capillary and cultured under a floating condition in Advanced MEM (GIBCO, 12492013) containing 10% FBS, 2 mM L-glutamine (GIBCO, 25030081), 150 µM ascorbic acid (Sigma-Aldrich, A4403), 1X penicillin/streptomycin (GIBCO, 15070063), and 55 µM 2-mercaptoethanol (GIBCO, 21985023) [*in vitro* differentiation medium for primates: P-IVD medium]. Medium change was performed every two days by transferring the cy reconstituted ovary into another well with freshly prepared culture medium. Cy reconstituted ovaries were analyzed at 3/6/9/12/15 w-ivc (Day 23/44/65/86/107). The human reconstituted ovary was generated from human fetal ovarian cells at 11 weeks of gestation and cultured under the same conditions. In the Examples where steroid hormones (progesterone (Sigma, P6149), cortisol (also known as hydrocortisone) (Sigma, H0135), dehydroepiandrosterone (also known as trans-dehydroandrosterone) (Sigma, D4000), and sulfate conjugate thereof, dehydroepiandrosterone sulfate (Cayman chemical, 15873)) and/or retinoic acid (Sigma, R2625) were added, the compounds were added to the medium from the start of *in vitro* culture.

### (Xenotransplantation experiment)

Cy reconstituted ovaries generated by the culture method described above were transplanted under the kidney capsule of immunodeficient KSN/Slc mice. Transplantation procedures were performed under mixed anesthesia (midazolam, medetomidine, and butorphanol), and the cy reconstituted ovaries were placed beneath the kidney capsule by narrow glass capillaries. Histological and SC3-seq analyses were performed at 3/6/9/12/15 w-ptp (post-transplantation).

### (Isolation of mouse gonadal/ovarian cells)

To isolate mouse gonadal/ovarian cells at embryonic day (E) 11.5 for the bulk RNA-seq analysis, VR females were crossed with the Dppa3(Stella)-EGFP^{Tg/Tg} males. Noon of the day when a copulation plug was identified was designated as E0.5. The sex of embryos at E11.5 was determined by sex-specific PCR for the Ubel locus. To isolate mouse gonadal/ovarian cells at E12.5-postnatal day (P)1, ICR females were crossed with the VR males, and the sex of embryos was determined by the gross appearance. The gonads/ovaries were dissociated into single cells by incubating with 0.05% trypsin-EDTA (GIBCO, 15400-054) for 10 min at 37°C, and were quenched with an equal volume of DMEM containing 10% FBS. Large clumps of cells were removed using a nylon cell strainer (FALCON, 352350), and cells were centrifuged at 200 rcf for 5 min. The cell pellet was re-suspended with DMEM/F12 medium (GIBCO, 1130-082) containing 0.1% BSA fraction V (Gibco, 15260037), and sorted into VR⁺ germ and VR⁻ somatic cells with a fluorescence-activated cell sorter (BD Biosciences, FACS Aria III).

### (10X Genomics single-cell RNA-seq)

Dissociated cells from cy fetal ovaries and cultured cy reconstituted ovaries were stained with DRAQ7 to distinguish dead cells. All DRAQ7⁻ live cells were collected by using a fluorescence-activated cell sorter (BD Biosciences, FACS Aria III). Subsequent 10X scRNA-seq library preparation (10X Genomics, Single Cell 3' Kit v. 3 and v. 3.1) and sequencing on a NovaSeq 6000 platform (Illumina) were performed following the manufacturers' instructions. Raw files were processed with the "mkfastq" command in Cell Ranger (v. 5.0.1) and mapped to the cynomolgus monkey reference genome (MacFas5.0 for cynomolgus monkeys and GRCh38.p12 for humans).

### (Analysis for cy scRNA-seq data (10X))

The gene-barcode matrices were analyzed with the "Seurat" R package (v. 3.2.2) (Satija R, et al., Nat Biotechnol, 33: 495-502 (2015)) following the online tutorials (https://satijalab.org/seurat/index.html). The low-quality cells, putative doublets/multiplets, and putative stripped nuclei were excluded for downstream analyses (Table 2). The raw counts for each cell were divided by their size factors calculated by the "scran" R package (v. 1.14.1), and then log-transformed [log2(size-scaled (ss) UMI+1)] for further analysis. Doublet cells were removed using the "Scrublet" python package (v. 0.2.1) following the previously reported procedures (Wolock SL, et al., Cell Syst, 8: 281-291 e9 (2019)). The threshold for doublet estimation was determined based on the bimodal distribution calculated by default parameters. Batch correction was then performed using the "RunFastMNN" function in the Seurat R package with the default setting. The Seurat "RunUMAP" function was used to characterize and visualize cell clusters. The resolution of the "FindClusters" function was set at 0.5 for the overall cell analyses and 0.8 for germ/granulosa cell analyses. Clusters were annotated by checking the expression pattern of previously characterized marker genes in human and mouse fetal ovarian cells. The sources and identification numbers of other R or Python packages are described in Table 3.

**[Table 3-1]**

| Reagents and Tools Table | | |
|---|---|---|
| **Reagent/Resource** | **Reference or Source** | **Identifier or Catalog Number** |
| **Experimental Models** | | |
| SIc:ICR (*M*. *musculus*) | Japan SLC | N/A |
| KSN/SIc (*M. musculus*) | Japan SLC | N/A |
| *Vasa*-RFP Tg (*M*. *musculus*) | B6C3(Cg)-Tg(Ddx4-RFP,neo)1Tne | RBRC03449 |
| *Dppe3*(*Stella*)-EGFP *Tg* (*M*. *musculus*) | Payer et al.,2006., Seki et al., 2007 | N/A |
| Cynomolgus monkey (*M*. *fascicularis*) | China / Philippines / Vietnam / Cambodia | N/A |

| **Antibodies** | | |
|---|---|---|
| Mouse anti-AP-2y/TFAP2C, monoclonal | Santa Cruz Biotechnology | Cat # sc-12762 |
| Rabbit anti-Cleaved Caspase-3, polyclonal | Cell Signaling Technology | Cat # #9661 |
| Rabbit anti-Cleaved PARP, monoclonal | Cell Signaling Technology | Cat # #5625 |
| Rabbit anti-CLGN, polyclonal | Atlas Antibodies | Cat # HPA048761 |
| Mouse anti-DAZL, monoclonal | Santa Cruz Biotechnology | Cat # sc-390929 |
| Mouse anti-DDX4, monoclonal | Abcam | Cat # ab27591 |
| Rabbit anti-DDX4, polyclonal | Abcam | Cat # ab13840 |
| Goat anti-VASA/DDX4, polyclonal | R&D Systems | Cat # AF2030 |
| Mouse anti-Dmc1, monoclonal | Santa Cruz Biotechnology | Cat # sc-53269 |
| Rabbit anti-FIGLA, polyclonal | Abcam | Cat # ab173725 |
| Goat anti-FOXL2, polyclonal | Novus Biologicals | Cat # NB100-1277 |
| Rabbit anti-FOXL2, monoclonal | Abcam | Cat # ab246511 |
| Rabbit anti-Ki67, polyclonal | Abcam | Cat # ab15580 |
| Rabbit anti-Laminin, polyclonal | Abcam | Cat # ab11575 |
| Rabbit anti-Leiomodin-3, polyclonal | Abcam | Cat # ab122305 |
| Rabbit anti-NALP5/NLRP5, polyclonal | Abcam | Cat # ab185679 |
| Goat anti-Nanog, polyclonal | R&D Systems | Cat # AF1997 |
| Mouse anti-NOBOX, monoclonal | Novus Biologicals | Cat # NBP2-46193 |
| Mouse anti-p63/TP63, monoclonal | Abcam | Cat # ab735 |
| Rabbit anti-PAX6, monoclonal | Abcam | Cat # ab195045 |
| Rat anti-Podoplanin/PDPN, monoclolnal | BioLegend | Cat # 337001 |
| Mouse anti-Oct3/4/POU5F1, monoclonal | Santa Cruz Biotechnology | Cat # sc-5279 |
| Rabbit anti-SCP1, polyclonal | Novus Biologicals | Cat # NB300-228 |

**[Table 3-2]**

| | | |
|---|---|---|
| Rabbit anti-SCP3, polyclonal | Abcam | Cat # ab15093 |
| Rabbit anti-Stra8, polyclonal | Abcam | Cat # ab217380 |
| Rabbit anti-ZGLP1, polyclonal | Atlas Antibodies | Cat # HPA049855 |
| Rabbit anti-ZP3, polyclonal | Atlas Antibodies | Cat # HPA054061 |
| Rabbit anti-gamma H2AX, polyclonal | Novus Biologicals | Cat # NB100-2280 |
| Donkey anti-Rat IgG (H+L) Highly Cross-Adsorbed Secondary Antibody, Alexa Fluor 488 | Invitrogen | Cat # A21208 |
| Donkey anti-Rabbit IgG (H+L) Highly Cross-Adsorbed Secondary Antibody, Alexa Fluor 488 | Invitrogen | Cat # A21206 |
| Donkey anti-Mouse IgG (H+L) Highly Cross-Adsorbed Secondary Antibody. Alexa Fluor 488 | Invitrogen | Cat # A21202 |
| Donkey anti-Goat IgG (H+L) Cross-Adsorbed Secondary Antibody, Alexa Fluor 488 | Invitrogen | Cat # A11055 |
| Donkey anti-Rabbit IgG (H+L) Highly Cross-Adsorbed Secondary Antibody, Alexa Fluor 568 | Invitrogen | Cat # A10042 |
| Donkey anti-Mouse IgG (H+L) Highly Cross-Adsorbed Secondary Antibody, Alexa Fluor 568 | Invitrogen | Cat # A10037 |
| Donkey anti-Goat IgG (H+L) Cross-Adsorbed Secondary Antibody, Alexa Fluor 568 | Invitrogen | Cat # A11057 |
| Donkey anti-Rabbit IgG (H+L) Highly Cross-Adsorbed Secondary Antibody, Alexa Fluor 647 | Invitrogen | Cat # A31573 |
| Donkey anti-Mouse IgG (H+L) Highly Cross-Adsorbed Secondary Antibody, Alexa Fluor 647 | Invitrogen | Cat # A31571 |
| Donkey anti-Goat IgG (H+L) Cross-Adsorbed Secondary Antibody, Alexa Fluor 633 | Invitrogen | Cat # A21082 |

| **Oligonucleotides and sequence-based reagents** | | |
|---|---|---|
| PCR primers | This study | Table EV8 |
| **Chemicals, enzymes and other reagents** | | |
| LEUPLIN FOR INJECTION 1.88mg. | TAKEDA Pharmaceutical | N/A |
| uFSH INJECTION | ASKA Pharmaceutical | N/A |
| GONATROPIN | ASKA Animal Health | N/A |
| DMEM, high glucose, pyruvate, no glutamine | GIBCO | 10313021 |

**[Table 3-3]**

| | | |
|---|---|---|
| DMEM/F12 | GIBCO | 11330057 |
| MEM α, nucleosides | GIBCO | 12571063 |
| Advanced MEM | GIBCO | 12492013 |
| CMRL Medium, no glutamine | GIBCO | 11530037 |
| DMEM/F-12, no glutamine | GIBCO | 21331020 |
| IMDM | GIBCO | 12440053 |
| KnockOut DMEM | GIBCO | 10829018 |
| McCoy's 5A (Modified) Medium | GIBCO | 16600082 |
| MesenPRO RS Medium | GIBCO | 12746012 |
| StemPro-34 SFM (1X) | GIBCO | 10639011 |
| Waymouth's Medium | GIBCO | 11220035 |
| Glasgow's MEM (GMEM) | GIBCO | 11710035 |
| Advanced RPMI 1640 | GIBCO | 12633012 |
| Fetal bovine serum | GIBCO | 10437028 |
| BSA fraction V | GIBCO | 15260037 |
| Penicillin-Streptomycin (5,000 U/mL) | GIBCO | 15070063 |
| MEM Non-Essential Amino Acids Solution (100X) | GIBCO | 11140-050 |
| Sodium Pyruvate (100 mM) | GIBCO | 11360070 |
| L-Glutamine (200 mM) | GIBCO | 25030081 |
| L-Ascorbic Acid | Sigma-Aldrich | A4403 |
| 2-Mercaptoethanol | GIBCO | 21985023 |
| Y-27632 dihydrochloride | TOCRIS | 1254 |
| Trypsin-EDTA (0.5%), no phenol red | GIBCO | 15400054 |
| TryPLE Express | GIBCO | 12604021 |
| Accumax | Innovative Cell Technologies | AM105 |
| Deoxyribonuclease I | MERCK | DN25 |
| CELLBANKER 1 plus | ZENOGEN PHARMA | CB021 |
| Human collagen type I | Millipore | CC050 |
| Human collagen type III | Corning | 354244 |
| Human collagen type IV | Corning | 354245 |
| Human collagen type V | Corning | 354246 |
| Human collagen type VI | Corning | 354261 |

**[Table 3-4]**

| | | |
|---|---|---|
| Human fibronectin | Corning | 354008 |
| iMatrix | Nippi | 892014 |
| Laminins (111/121/211/221/411/421/3321521) | VERITAS | BLA-LNKT-0201 |
| Matrigel Growth Factor Reduced | Corning | 356230 |
| Paraformaldehyde | Sigma-Aldrich | 158127 |
| Hematoxylin | Fujifilm | 131-09665 |
| Eosin | Fujifilm | 058-00062 |
| OCT Compound | Sakura Finetek | 4583 |
| HistVT One | Nacalai Tesque | 06380-76 |
| Bovine serum albumin | Sigma-Aldrich | A7030 |
| Normal donkey serum | Jackson Immuno Research Laboratories | 017-000-121 |
| DAPI | Wako | 342-07431 |
| VECTASHIELD | Vector Laboratories | H-1000 |
| **Software** | | |
| FV10-ASW | Olympus | N/A |
| FACSDiva | BD Biosciences | N/A |
| DAVID (v6.8; GO analysis) | N/A | https://david.ncifcrf.gov/tools.jsp |
| Cell Ranger (version 5.0.1) | N/A | https://github.com/10XGenomics/cellranger |
| R (version 3.6.3) | N/A | https://www.R-project.org/ |
| Python (version 3.7.7) | N/A | https://www.python.org |
| Seurat (version 3.2.2; 10X analysis) | Stuart et al., 2019 | https://github.com/satijalab/seurat |
| SeuratWrappers (version 0.1.0) | N/A | https://github.comlsatijalablseurat-wrappersl |
| scran (version 1.14.1) | Lun et al., 2016 | https://bioconductor.org/packages/release/bio c/html/scran.html |
| Monocle3 (version 0.2.1) | Cao et al., 2019 | http://cole-trapnell-lab.github.io/monocle-release/ |
| ComplexHeatmap (version 2.6.2) | Gu et al., 2016 | https://github.com/jokergoo/ComplexHeatma p |
| LTLA/batchelor (version 1.2.1) | Haghverdi et al., 2018 | https://rdrr.io/github/LTLA/batchelor/ |
| Scrublet (version 0.2.1) | Wolock et al., 2019 | https://github.com/swolock/scrublet |
| scVelo (version 0.2.2) | Bergen et al., 2020 | https://github.com/theislab/scvelo |
| Scanpy (version 1.6.0) | Wolf et al., 2018 | https://github.com/theislab/scanpy |

**[Table 3-5]**

| | | |
|---|---|---|
| R (version 4.0.2) | N/A | https://www.R-project.org/ |
| dendextend (version 1.15.1) | Galili et al., 2015 | https://CRAN.R-project.org/package=dendextend |
| amap (version 0.8-18) | N/A | https://CRAN.R-project.org/package=amap |
| pheatmap (version 1.0.12) | N/A | https://CRAN.R-project.org/package=pheatmap |
| Seurat (version 4.0.4; SC3-Seq analysis) | Stuart et al., 2019 | https://github.com/satijalab/seurat |
| **Other** | | |
| Nunclon Sphera-Treated U-Shaped-Bottom 96-well plates | NUNC | 174925 |
| Transwell-COL | Corning | 3495 |
| Preset VECELL | VECELL | PSVC12-10 |
| QIAamp DNA Mini Kit | QIAGEN | 51304 |
| Power SYBR Green PCR Master Mix | Applied Biosystems | 4367659 |
| CFX384 Touch Real-Time PCR Detection System | BIO-RAD | N/A |
| DNA High Sensitivity Reagent Kit | PerkinElmer | CLS760672 |
| Chromium Single Cell 3' GEM, Library & Gel Bead Kit | 10X Genomics | 1000092 |
| (version 3) | | |
| Next GEM single cell 3' Library & Gel beads kit (version 3.1) | 10X Genomics | 1000128 |
| Chromium Single Cell B Chip Kit | 10X Genomics | 1000074 |
| Chromium Next GEM single cell G chip kit | 10X Genomics | 1000120 |
| Mastercycler nexus | Eppendorf | 6331000033 |
| NucleoSpin RNA XS | Macheray-Nagal | 740902 |
| NextSeq 500/550 | Illumina | N/A |
| NovaSeq 6000 | Illumina | N/A |
| NextSeq 500/550 High-Output v2 Kit (75 cycles) | Illumina | FC-404-2005 |
| NovaSeq 6000 SP Reagent Kit (100 cycles) | Illumina | 20027464 |
| NovaSeq 6000 S1 Reagent Kit (100 cycles) | Illumina | 20012865 |
| Microtome | Leica | CX41 |
| Upright microscope | Olympus | BX53 |
| Inverted microscope | Olympus | IX71 |

**[Table 3-6]**

| | | |
|---|---|---|
| CCD camera | Olympus | DP70 |
| Cryostat | Leica | CM1850 |

### (Analysis for cy germ cells (10X))

Cells in the DDX4⁺ germ cell cluster were further analyzed and divided into 13 clusters using the "FindClusters" function. After removing two small clusters consisting of putative doublets/multiplets that co-expressed the markers of other cell types, 11 sub-stages of meiotic prophase I were defined by the expression pattern of key germ cell markers as "mitotic 1/2/3," "pre-leptotene 1/2/3," "leptotene," "zygotene," "pachytene 1/2," and "unclassified." Cells expressing GDF9/NOBOX/ZP3 were manually selected as another cluster defined as "Diplotene." The "FindVariableFeatures (selection.method= "vst," nfeatures=2,000)" function in the Seurat R package was performed across the 11 sub-stages (mitotic-diplotene) to identify highly variable genes (HVGs) during cy female germ cell development, and 2,000 HVGs were detected. Among them, excluding genes with low expression levels [cluster average of log(ssUMI+1) ≤0.5: 519 genes], 1,481 genes were used for heatmap visualization, GO enrichment analysis (see below), and calculation of the correlation coefficients between each meiotic sub-stage (Figs. 6H and I). The pseudotime trajectory analysis was performed using the "Monocle 3" R package (v. 0.2.1). Cy germ cells from cultured reconstituted ovaries at 12/15 w-ivc were also processed in the same manner.

### (Analysis for cy granulosa cells (10X))

Cells in the FOXL2⁺ granulosa cell cluster were further analyzed. The count matrices for spliced and unspliced transcripts were created by running the commands "count--include-introns" and "count" in Cell Ranger (v. 5.0.1). The resolution of the "FindClusters" function was set at 0.8, and the cells were divided into 12 clusters after removing two small clusters consisting of putative doublets/multiplets that co-expressed the markers of other cell types. RNA velocity analysis was performed using the "scvelo.pp.moments(n_pcs=30, n_neighbors=30)" and "scvelo.tl.velocity(mode='stochastic')" functions in the scVelo python package (v. 0.2.0) (Bergen V, et al., Nat Biotechnol, 38: 1408-1414 (2020)). Partition-based graph abstraction (PAGA) graph (Wolf FA, et al., Genome Biol, 20: 59 (2019)) was computed on the clusters annotated in Fig. 8A using the "scanpy.tl.paga" function from the Scanpy (v. 1.2.2) in Python (v. 3.7.7) following the online tutorials (https://scanpy-tutorials.readthedocs.io/en/latest/paga-paul15.html), and the threshold for connection of clusters was set to 0.03. The "FindVariableFeatures (selection.method= "vst," nfeatures=1,500)" function in the Seurat R package was used to identify HVGs among the 12 clusters, and 1,500 HVGs were detected. Among them, excluding genes with low expression levels [cluster average of log(ssUMI+1) ≤0.5: 712 genes], 788 genes were used for heatmap visualization and GO enrichment analysis (see below) (Fig. 8D). Cy granulosa cells from cultured reconstituted ovaries at 12/15 w-ivc were also processed in the same manner.

### (SC3-seq for cy/human germ cells)

Dissociated germ cells from *in vivo* cy/human fetal ovaries and cy/human reconstituted ovaries were manually picked up using glass capillaries under a stereomicroscope (Leica, S8 APO). The following cDNA synthesis and amplification methods were previously described (Kurimoto K, et al., Nucleic Acids Res, 34: e42 (2006), Kurimoto K, et al., Nature protocols, 2: 739-52 (2007), Nakamura T, et al., Nature, 537: 57-62 (2016), Nakamura T, et al., Nucleic Acids Res, 43: e60 (2015)). The quality of the amplified cDNA was measured by a SYBR real-time PCR method targeting the PPIA, GAPDH, NANOG, DDX4, DAZL, SYCP3, SYCP1, ZP3, and FOXL2 genes. The primer sequences are listed in Table 4. The SC3-seq libraries were constructed as described previously (Nakamura T, et al., Nature, 537: 57-62 (2016), Nakamura T, et al., Nucleic Acids Res, 43: e60 (2015), Okamoto I, et al., Science, 374: eabd8887 (2021)), and the sequencing was performed on the Illumina NextSeq 500/550 platform with a NextSeq 500/550 high output kit v. 2 (75 cycles) (FC-404-2005; Illumina).

**[Table 4]**

| **Primers used in the Examples** | | | | |
|---|---|---|---|---|
| **Cynomolgus monkey (Macaca *fascicularis*)** | | | | |
| **Gene name** | **sense** | **SEQ ID No** | **anti-sense** | **SEQ ID No** |
| *GAPDH* | AACAGGGTGGTGGACCTCAT | 1 | TTCCTCTTGTGCTCTCGCTG | 2 |
| *NANOG* | TGTTCCGGTTTCCATTATGCC | 3 | TAGGCTCCAACCATACTCCA | 4 |
| *PPIA* | ACAGGTCCTGGCATCTTGTC | 5 | CTCAGTCTTGGCAGTGCAGA | 6 |
| *DDX4* | CTTCTTCAGGGGGCTTAGACA | 7 | ACTCAAGTAAAAATGAGACCCAGT | 8 |
| SCP1 | AGGCCCCTTCATCTCTAACAAC | 9 | TTTAGCAATTACAGCCCAACGG | 10 |
| *ZP3* | GTCCAGGTCTGCTTCTCGTAAC | 11 | GGCCCACTGCTCTACTTCATAG | 12 |
| *FOXL2* | CCACCTTGGGAAGGAGAAATGT | 13 | TTGGTCCCCCAAACAAGTTACA | 14 |

| **Human *(Homo sapiens)*** | | | | |
|---|---|---|---|---|
| **Gene name** | **sense** | **SEQ ID No** | **anti-sense** | **SEQ ID No** |
| *GAPDH* | ACAAGAGGAAGAGAGAGACCCT | 15 | TCTACATGGCAACTGTGAGGAG | 16 |
| *NANOG* | AGAGGTCTCGTATTTGCTGCAT | 17 | AAACACTCGGTGAAATCAGGGT | 18 |
| *DAZL* | GAATTCCCAAGAGGGCTTTATTACAC | 19 | TGATATGTGTCAGAATGTTATAGCACC | 20 |
| *DDX4* | TTGCAGTTACTGATACAAATGGTGTT | 21 | TAAACATGTCTAAGCCCCCTAAAGAA | 22 |
| *SCP1* | AGGCCCCTTCATCTCTAACAAC | 23 | TTTAGCAATTACAGCCCAACGG | 24 |
| *ZP3* | TAACAAAGGTGACTGTGGCACT | 25 | CCACTGCTCTACTTCATGGTCA | 26 |
| *FOXL2* | CCTTCAAAAAGCTGTGTTCAGAGTTA | 27 | TTCAGATAGGGAGAGGGTGAAACTT | 28 |

### (Bulk RNA-seq analysis for mouse gonadal/ovarian cells)

The mouse gonadal/ovarian cells were lysed and the total RNAs were purified using a NucleoSpin RNA XS (Takara Bio, U0902A) according to the manufacturer's instructions. 1 ng of total RNAs from each sample was used for the synthesis and amplification of cDNAs. The cDNA synthesis and library construction were as described previously (Ishikura Y, et al., Cell reports, 17: 2789-2804 (2016), Nakamura T, et al., Nucleic Acids Res, 43: e60 (2015), Okamoto I, et al., Science, 374: eabd8887 (2021)). The sequencing was performed on the Illumina NextSeq 500/550 platform with a NextSeq 500/550 high output kit v. 2 (75 cycles) (FC-404-2005; Illumina).

### (Mapping reads of bulk RNA-seq/SC3-seq and conversion to gene expression levels)

The genome sequence and transcript annotation (GRCm38.p6 for mice, GRCh38.p12 for humans, and MacFas5.0 for cynomolgus monkeys) were downloaded from the NCBI ftp site. Bulk RNA-seq/SC3-seq read only the 3' end of the transcripts, so that the expression levels were calculated as genes (Entrez genes) but not mRNAs. Read trimming, mapping and estimation of expression levels were performed as described previously (Ishikura Y, et al., Cell reports, 17: 2789-2804 (2016), Nakamura T, et al., Nature, 537: 57-62 (2016)).

### (Orthologous genes among three species)

For the cross-species comparison analyses for SC3-seq and 10X scRNA-seq data, the lists of orthologous genes among the three species were retrieved from Ensembl BioMart (Ensembl 97: Jul 2019). One-to-one (1-1) orthologous genes between human and cy (16,734 genes) were used for the cross-species integration of SC3-seq data. One-to-one-to-one (1-1-1) orthologous genes across three species (15,007 genes) were used for the cross-species integration of 10X data. Since the orthologous gene list from Ensembl BioMart was relatively incomplete, a genomic coordinate comparison list was made using the LiftOver tool as previously described (Nakamura T, et al., Nature, 537: 57-62 (2016), Sasaki K, et al., Cell Stem Cell, 17: 178-94 2015). The list of orthologous genes is omitted.

### (SC3-seq data analysis for cy/human germ cells)

The SC3-seq data analysis was performed using the R software (v. 4.0.2). The read counts were normalized to 200,000 reads, and then log-transformed (log2 (normalized read counts+1)). "All expressed genes" were defined as genes whose log2(normalized read counts+1) values were > 2 [greater than about 10-20 copies per cell] in at least one sample (Nakamura T, et al., Nucleic Acids Res, 43: e60 (2015)). Unsupervised hierarchical clustering (UHC) was performed using the "hclust" function with Euclidean distances and Ward's method (ward.D2). The principal component analysis (PCA) was performed using the "prcomp" function without scaling.

For the cross-species analysis between monkeys and humans, 1-1 orthologous genes between humans and monkeys were used, and data were processed with the "Seurat" R package (v. 4.0.4). The cy SC3-seq dataset was integrated with the human dataset using the Seurat CCA algorithm, i.e., "FindIntegrationAnchors" (parameter "anchor.features" = 5,000) and "IntegrateData" functions with the default settings. Integrated germ cells were divided into four clusters using the Seurat "FindClusters" function (parameter "resolution" = 0.4). PCA was performed on the corrected count matrix obtained after adjusting for species differences. Gene expression levels were compared between two consecutive clusters, and DEGs were defined as those with greater than 2-fold change between clusters and an average gene expression level [log2(normalized read counts + 1)] greater than 3.

### (Gene ontology enrichment analysis)

Gene ontology (GO) enrichment analysis for HVGs/DEGs was performed by using the DAVID web tool (Huang da W, et al., Nature protocols, 4: 44-57 (2009)). Since the annotation of *Macaca fascicularis* genes was relatively incomplete, human annotation corresponding to that of cynomolgus monkeys was used for GO analysis. Missing genes in the 1-1 orthologous gene list from Ensembl BioMart were complemented by the orthologous gene list produced with the LiftOver tool.

### (Cross-species analysis among three animal species using 10X public datasets)

To perform the cross-species analysis among humans, monkeys, and mice, a 10X scRNA-seq dataset for human *in vivo* fetal germ cells (Chitiashvili T, et al., Nat Cell Biol, 22: 1436-1446 (2020)) and three datasets for mouse *in vivo* germ cells (Ge W, et al., Cell Mol Life Sci, 78: 695-713 (2021), Niu W, and Spradling AC, Proc Natl Acad Sci USA, 117: 20015-20026 (2020)) were retrieved. Downloaded raw files were processed with the "mkfastq" command in Cell Ranger and mapped to the reference genome sequence (GRCm38.p6 for mice and GRCh38.p12 for humans). The low-quality cells and putative doublets were excluded for downstream analyses. Since the thresholds for these cells depend on the quality of each dataset (e.g., the reagent version and the reference genome used), the number of detected genes, UMIs, and mitochondrial genes in each dataset were finely assessed to define the thresholds. Each dataset was processed in the same manner as described above for the analysis of cy 10X scRNA-seq data, and DDX4/Ddx4⁺ germ cell clusters were extracted for the following cross-species comparison analysis. Our cy/human 10X scRNA-seq datasets were integrated with the four public datasets. Each dataset was size-scaled to 10,000 UMIs (ss10kUMI) and processed by using the CCA algorithm, i.e., "FindIntegrationAnchors" (parameter "anchor.features" = 2,000) and "IntegrateData" functions in the "Seurat" R package (v. 3.2.2). PCA was performed on the corrected count matrix obtained after adjusting for species differences, and UMAP visualization was performed for 30 dimensions using the "RunUMAP" function. Integrated germ cells were divided into 15 clusters using the Seurat "FindClusters" function (parameter "resolution" = 0.8). Each cluster was defined by the expression pattern of key germ cell markers as "mitotic 1/2/3/4," "pre-leptotene 1/2/3," "leptotene 1/2/3," "zygotene 1/2," "pachytene 1/2," or "diplotene." To confirm the accuracy of the merged clusters, a pseudotime trajectory analysis was performed on each dataset using the "Monocle 3" R package (v. 0.2.1). Among 1-1-1 orthologous genes complemented with the LiftOver tool, common HVGs (237 genes) were obtained across three datasets/species, and Pearson correlation coefficients of the average HVGs expression levels between the meiotic sub-stages of the three animal species were calculated.

### (Conserved and primate-specific variable genes during female meiosis)

Among the DEGs identified by the cross-species comparison analysis with SC3-seq analysis as described above, the commonly variable 1-1 orthologous genes were defined as the genes conserved across humans and monkeys. The clusters defined in the 10X cross-species analysis were used to verify the average expression levels of these genes in 10X datasets from three animal species. Genes that varied identically in mice were selected manually as genes conserved among the three species (130 genes; Fig. 12A), and genes that showed no variation in expression in mice were selected as genes that varied specifically in primates (83 genes; Fig. 10F).

### (Chromosomal expression ratio analysis)

For bulk RNA-seq/SC3-seq data, the autosome (chromosome (chr.) 10 for humans, chr. 15 for monkeys, and chr. 3 for mice): total autosome ratio and the chr. X: total autosome ratio (X:A ratio) were calculated according to the method described previously (Okamoto I, et al., Science, 374: eabd8887 (2021)). Briefly, 75th-percentile log2(normalized read counts+1) values of the expressed genes [the maximum log2(normalized read counts +1) values > 2, among the cells analyzed] on the autosomes and chr. X in individual samples/cells were calculated and used as representative expression values in the samples/cells. 19,307/811/728 genes on total autosome/Chr.10/Chr.X for humans, 20,576/849/841 genes on total autosome/Chr.15/Chr.X for monkeys, and 12,399/618/503 genes on total autosome/Chr.3/Chr.X for mice were used.

For 10X scRNA-seq data, the average log2(ss10kUMI+1) values of all expressed genes, which were expressed in more than two cells, on the autosome (chr. 10 for humans, chr. 15 for monkeys, and chr. 3 for mice), all autosomes, and chr. X were used for the calculation of the autosome:A and X:A ratio. 17,190/682/725 genes on total autosome/Chr.10/Chr.X for humans (Chitiashvili T, et al., Nat Cell Biol, 22: 1436-1446 (2020)), 18,066/736/791 genes on total autosome/Chr.15/Chr.X for monkeys, and 17,698/896/744 genes on total autosome/Chr.3/Chr.X for mice (Niu W, and Spradling AC, Proc Natl Acad Sci USA, 117: 20015-20026 (2020)) were used.

### (Statistical analysis)

All statistical analyses were performed using R software (v. 4.0.2). The Tukey-Kramer tests and T-tests were performed with the "TukeyHSD" and "t.test" functions with the default settings.

### (Data and code availability)

The accession numbers for the data in this study: GSE194266 (the GEO database). The R and Python code to reproduce the analyses and figures are available on request.

### INDUSTRIAL APPLICABILITY

By using the ovarian follicles induced by the present invention, it is possible to observe the maturation process of female germ cells of primates *in vitro,* which is useful for understanding the mechanism of germ cell development. Thus, the present invention provides an important foundation for both biology and medicine.

The present application claims priority based on Japanese Patent Application No. 2022-117665 filed in Japan (filing date: July 25, 2022), the disclosure of which is incorporated herein in its entirety by reference.

## Claims

1. A method for producing oocytes or ovarian follicles, or a reconstituted ovary containing oocytes or ovarian follicles, comprising a step of culturing a reconstituted ovary of a primate under a floating culture condition.

2. The method according to claim 1, wherein the reconstituted ovary is constructed by aggregating primate germ cells and primate fetal ovarian somatic cells.

3. The method according to claim 2, wherein the germ cells and the fetal ovarian somatic cells are prepared by dissociation from a fetal ovary of the primate through enzymatic treatment.

4. The method according to claim 2 or 3, wherein the germ cells are at least one type selected from the group consisting of oogonia, primordial germ cells, and primordial germ cell-like cells.

5. The method according to any one of claims 2 to 4, wherein at least either one of the germ cells and the ovarian somatic cells are derived from pluripotent stem cells.

6. The method according to any one of claims 1 to 5, wherein the culture is carried out under the floating culture condition for 9 weeks or more.

7. The method according to any one of claims 1 to 6, wherein the culture under the floating culture condition is a culture in a medium containing L-glutamine, ascorbic acid, and 2-mercaptoethanol.

8. The method according to claim 7, wherein the medium further contains retinoic acid.

9. The method according to claim 7, wherein the medium further contains one or more selected from the group consisting of progesterone, cortisol, dehydroepiandrosterone, and dehydroepiandrosterone sulfate.

10. The method according to any one of claims 1 to 9, wherein the primate is a human.

11. Oocytes, ovarian follicles, or a reconstituted ovary produced by the method according to any one of claims 1 to 10.

12. A method for producing an ovum, comprising a step of maturing the oocytes according to claim 11.

13. A kit for producing oocytes or ovarian follicles, or a reconstituted ovary containing oocytes or ovarian follicles, comprising a culture vessel for floating culture, L-glutamine, ascorbic acid, and 2-mercaptoethanol.

14. The kit according to claim 13, further comprising a basal medium.

15. The kit according to claim 13 or 14, further comprising one or more selected from the group consisting of retinoic acid, progesterone, cortisol, dehydroepiandrosterone, and dehydroepiandrosterone sulfate.
